# EUROPEAN PATENT APPLICATION

(11) **EP 4 803 546 A1**
(43) Date of publication of application: **09.09.2026**
(21) Application number: 24883771.8
(22) Date of filing: 20.03.2024
(51) Int. Cl.: C07K 16/46, C07K 16/28, C07K 16/18, C07K 16/00, A61K 39/395, A61P 35/00, A61P 35/02

(54) **MULTIPLE ANTIBODIES BINDING TO HLA-A24/PRAME COMPLEX AND USE THEREOF**

(30) Priority: 31.10.2023 CN 202311429311
(71) Applicant: Beijing Wisdomab Biotechnology Co., Ltd, Beijing 101111 (CN); Genrix(Shanghai) Biopharmaceutical Co., Ltd., Shanghai 201203 (CN); Chongqing Genrix Biopharmaceutical Co., Ltd., Chongqing 401319 (CN)
(72) Inventor: FU, Xinlei, Beijing 101103 (CN); LIU, Zhigang, Beijing 100039 (CN); ZHOU, Xiaowei, Beijing 100039 (CN); HU, Junjie, Beijing 100176 (CN); ZHANG, Suhua, Beijing 102600 (CN); LIU, Yulan, Beijing 100176 (CN); HAO, Xiaobo, Beijing 100163 (CN)
(74) Representative: Richly & Ritschel Patentanwälte PartG mbB
(86) International application number: PCT/CN2024/082739
(87) International publication number: WO 2025/091756

(57) **Abstract**

Provided are a bispecific antibody comprising a first antigen-binding fragment that binds to an HLA-A24/PRAME complex and a second antigen-binding fragment that binds to an activated T-cell antigen, a single-domain antibody that binds to an HLA-A24/PRAME complex, and a pharmaceutical composition comprising the bispecific antibody or the single-domain antibody. Also provided is the use of the bispecific antibody or the single-domain antibody in medicine and biology.

## Description

### CROSS REFERENCE OF RELATED APPLICATION

The present application claims priority to Chinese Patent Application No. 202311429311.5, filed on October 31, 2023, which is incorporated herein by reference in its entirety.

### TECHNICAL FIELD

The present invention pertains to the field of genetic engineering and biomedicine, and more particularly, relates to a bispecific antibody that binds to an HLA-A24/PRAME complex, a single-domain antibody that binds to an HLA-A24/PRAME complex, and uses of the bispecific antibody and the single-domain antibody.

### BACKGROUND

Most of the proteins in the human proteome are intracellular, and some short peptides produced by degradation of the intracellular proteins in proteasomes can be presented on the cell surface by major histocompatibility complex (MHC) molecules, making them potential targets for cancer immunotherapy. Antibodies against these MHC/peptide complexes are developed to function like TCRs in recognizing the MHC/peptide complexes. These antibodies are referred to as T cell receptor mimics (TCRm) or TCR-like antibodies.

Preferentially Expressed Antigen in Melanoma (PRAME) belongs to the cancer/testicular antigen (CTA) gene family, and is expressed at a very low level in normal adult tissues other than the testis, but at a high level in various cancer cells ^{[1]}. The PRAME gene is located on the reverse strand of chromosome 22 (22q11.22), is about 12 kilobases in length, and contains a leucine-rich repeat domain ^{[2]}. It has been reported that the PRAME gene is hypermethylated in normal tissues; however, this gene is hypomethylated in most malignant cells ^{[3]}.

PRAME is highly expressed in 88% of primary melanoma tissues and 95% of metastatic melanoma tissues ^{[1]}. In addition to melanoma, PRAME is also widely expressed in many solid cancers, such as head and neck cancer, breast cancer, renal cell carcinoma, and non-small cell lung cancer ^{[4-7]}. PRAME is absent in normal hematopoietic tissues; however, some studies have found high expression levels of PRAME in acute and chronic leukemia, and Hodgkin lymphoma ^{[8-10]}. Furthermore, the expression level of PRAME is a prognostic biomarker for poor clinical outcomes in breast cancer and neuroblastoma ^{[11, 12]}.

PRAME is degraded into small-molecule polypeptides in proteasomes, and some of these peptides bind to MHC molecules to form complexes, which are presented on the cell surface. LYVDSLFFL corresponds to amino acids at positions 301-309 of the full-length PRAME protein, and is present on the cell surface in a complex with HLA-A24 ^{[13]}. This HLA-peptide complex provides a useful target for TCRm-based immunotherapeutic interventions.

Bispecific antibodies (BsAb) are a class of artificial antibodies that contain two different antigen-binding sites. Bispecific antibodies are widely used in the biomedical field, especially in tumor immunotherapy. A bispecific antibody targeting CD3 has one arm capable of binding CD3 in the TCR receptor complex on the T cell surface to provide the first signal for T cell activation, and the other arm targeting a tumor antigen. Bispecific antibodies can bring tumor cells and T cells closer together, activating T cells while directly killing tumor cells.

TCRm that binds to intracellular proteins like PRAME can serve as the part of a CD3 bispecific antibody that binds to a target antigen; such a bispecific antibody may be more attractive due to the restricted expression of the target antigen. Based on clinical needs, exploring and developing a bispecific antibody targeting PRAME-MHC complexes has important clinical significance.

### SUMMARY OF THE INVENTION

In a first aspect, the present application provides a bispecific antibody comprising a first antigen binding fragment that binds to an HLA-A24/PRAME complex and a second antigen binding fragment that binds to an activated T cell antigen.

In some embodiments of the first aspect, the bispecific antibody is capable of mediating T cell activation in response to HLA-A24⁺/PRAME⁺ tumor cells, and/or the bispecific antibody is capable of mediating killing of HLA-A24⁺/PRAME⁺ tumor cells by PBMCs.

In some embodiments of the first aspect, the first antigen-binding fragment comprises HCDR1 as set forth in SEQ ID NO: 1, HCDR2 as set forth in SEQ ID NO: 2, and HCDR3 as set forth in SEQ ID NO: 3; wherein the amino acid sequences of HCDRs are defined according to Kabat numbering scheme.

In some embodiments of the first aspect, the first antigen-binding fragment is in the form of a single-domain antibody.

In some embodiments of the first aspect, the first antigen-binding fragment comprises a monovalent or multivalent single-domain antibody that binds to the HLA-A24/PRAME complex.

In some embodiments of the first aspect, the second antigen-binding fragment comprises HCDR1 as set forth in SEQ ID NO: 6, HCDR2 as set forth in SEQ ID NO: 7, HCDR3 as set forth in SEQ ID NO: 8, LCDR1 as set forth in SEQ ID NO: 9, LCDR2 as set forth in SEQ ID NO: 10, and LCDR3 as set forth in SEQ ID NO: 11; wherein the amino acid sequences of HCDRs are defined according to Kabat numbering scheme.

In some embodiments of the first aspect, the second antigen-binding fragment is in the form of a single-chain variable fragment (scFv) or a Fab fragment.

In some embodiments of the first aspect, the first antigen-binding fragment comprises a divalent single-domain antibody that binds to the HLA-A24/PRAME complex; optionally, the first antigen-binding fragment comprises two heavy chain variable regions of monovalent single-domain antibodies that bind to the HLA-A24/PRAME complex.

In some embodiments of the first aspect, the first antigen-binding fragment comprises the amino acid sequence as set forth in SEQ ID NO: 4, 5, 14, or 15; and/or the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises the amino acid sequence as set forth in SEQ ID NO: 4; and the second antigen-binding fragment comprises a heavy chain variable region having the amino acid sequence as set forth in SEQ ID NO: 12 and a light chain variable region having the amino acid sequence as set forth in SEQ ID NO: 13; or the first antigen-binding fragment comprises the amino acid sequence as set forth in SEQ ID NO: 5; and the second antigen-binding fragment comprises a heavy chain variable region having the amino acid sequence as set forth in SEQ ID NO: 12 and a light chain variable region having the amino acid sequence as set forth in SEQ ID NO: 13; or the first antigen-binding fragment comprises the amino acid sequence as set forth in SEQ ID NO: 14; and the second antigen-binding fragment comprises a heavy chain variable region having the amino acid sequence as set forth in SEQ ID NO: 12 and a light chain variable region having the amino acid sequence as set forth in SEQ ID NO: 13; or the first antigen-binding fragment comprises the amino acid sequence as set forth in SEQ ID NO: 15; and the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen binding fragment and the second antigen binding fragment are linked via an Fc fragment of an antibody heavy chain constant region comprising a first Fc fragment and a second Fc fragment, wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407; wherein the amino acid positions of the antibody constant regions are determined according to EU numbering scheme.

In some embodiments of the first aspect, the first antigen binding fragment and the second antigen binding fragment are linked via an Fc fragment of an antibody heavy chain constant region comprising a first Fc fragment and a second Fc fragment, wherein the first Fc fragment and the second Fc fragment comprise the amino acid F at position 234, the amino acid E at position 235 and the amino acid S at position 331, respectively; wherein the amino acid positions of the antibody constant regions are determined according to EU numbering scheme.

In some embodiments of the first aspect, the first antigen-binding fragment and the second antigen-binding fragment are linked via an Fc fragment of an antibody heavy chain constant region comprising a first Fc fragment and a second Fc fragment, wherein one of the first Fc fragment and the second Fc fragment is linked to the first antigen-binding fragment and the other of the first Fc fragment and the second Fc fragment is linked to the second antigen-binding fragment.

In some embodiments of the first aspect, the bispecific antibody comprises a first arm that binds to the HLA-A24/PRAME complex and a second arm that binds to the activated T cell antigen, wherein the first arm comprises the amino acid sequence as set forth in SEQ ID NO: 35 or 36; and the second arm comprises the amino acid sequence as set forth in SEQ ID NO: 37.

In a second aspect, the present application provides a single-domain antibody that binds to an HLA-A24/PRAME complex, comprising HCDR1 as set forth in SEQ ID NO: 1, HCDR2 as set forth in SEQ ID NO: 2, and HCDR3 as set forth in SEQ ID NO: 3; wherein the amino acid sequences of HCDRs are defined according to Kabat numbering scheme.

In some embodiments of the second aspect, the single-domain antibody binds to the HLA-A24/PRAME complex at an epitope comprising one or more residues at positions 301-309 of PRAME as set forth in SEQ ID NO: 38.

In some embodiments of the second aspect, the single-domain antibody is in the form of a monovalent or multivalent single-domain antibody that binds to the HLA-A24/PRAME complex.

In some embodiments of the second aspect, the single-domain antibody is in the form of a bivalent single-domain antibody that binds to the HLA-A24/PRAME complex.

In some embodiments of the second aspect, the single-domain antibody comprises the amino acid sequence as set forth in SEQ ID NO: 4, 5, 14, or 15.

In a third aspect, the present application provides a nucleic acid molecule encoding the bispecific antibody of the first aspect or the single-domain antibody of the second aspect.

In a fourth aspect, the present application provides a pharmaceutical composition comprising the bispecific antibody of the first aspect or the single-domain antibody of the second aspect, and a pharmaceutically acceptable excipient, diluent or carrier.

In a fifth aspect, the present application provides use of the bispecific antibody of the first aspect, the single-domain antibody of the second aspect, or the pharmaceutical composition of the fourth aspect in the manufacture of a medicament for the prevention or treatment of an HLA-A24/PRAME-positive tumor.

In a sixth aspect, the present application provides use of the bispecific antibody of the first aspect or the single-domain antibody of the second aspect in the manufacture of a product for detecting HLA-A24/PRAME positive cells.

### BRIEF DESCRIPTION OF THE DRAWINGS

FIG. 1 shows the results demonstrating that the antibody against the HLA-A24/PRAME complex binds to the PRAME₃₀₁₋₃₀₉ (LYVDSLFFL) pulsed HT-29 cells.
FIG. 2 shows the results demonstrating that the bispecific antibody 2N83G3×IMCR and the humanized bispecific antibody 2N83G3-h5×IMCR mediate the activation of Jurkat-Dual cells via tumor cells.
FIG. 3 shows the results demonstrating that the humanized bispecific antibody 2N83G3-h5×IMCR mediates the killing of tumor cells via PBMCs.
FIG. 4 shows the results demonstrating that the humanized bispecific antibody 2N83G3-h5×IMCR mediates the activation of Jurkat-Dual cells via alanine-substituted peptide-pulsed HT-29 cells.
FIG. 5 shows the results demonstrating that the humanized bispecific antibody 2N83G3-h5×IMCR mediates the killing of HT-29 cells transfected with PRAME or a gene encoding similar peptide segment 8/11/12/16 via PBMCs.
FIG. 6 shows the results demonstrating that the humanized bispecific antibody 2N83G3-h5×IMCR mediates the killing of 293T cells transfected with PRAME or a gene encoding similar peptide segment 8/11/12/16 via PBMCs.
FIG. 7 shows the results demonstrating the antitumor activity of the humanized bispecific antibody 2N83G3-h5×IMC in PBMC-reconstituted mice models.

### DESCRIPTION OF SEQUENCE

SEQ ID NO: 1-3 shows the amino acid sequences of HCDR1, HCDR2 and HCDR3 of the heavy chain variable regions of *Llama*-derived single-domain antibodies N83G3 and N83G3-h5, respectively.

SEQ ID NO: 4 shows the amino acid sequence of the heavy chain variable region of *Llama-*derived single-domain antibody N83G3.

SEQ ID NO: 5 shows the amino acid sequence of the heavy chain variable region of the humanized single-domain antibody N83G3-h5.

SEQ ID NO: 6-8 shows the amino acid sequences of HCDR1, HCDR2 and HCDR3 of the heavy chain variable region of the anti-human CD3 antibody IMCR, respectively.

SEQ ID NO: 9-11 shows the amino acid sequences of LCDR1, LCDR2 and LCDR3 of the light chain variable region of the anti-human CD3 antibody IMCR, respectively.

SEQ ID NO: 12 shows the amino acid sequence of the heavy chain variable region of the anti-human CD3 antibody IMCR.

SEQ ID NO: 13 shows the amino acid sequence of the light chain variable region of the anti-human CD3 antibody IMCR.

SEQ ID NO: 14 shows the amino acid sequence of the tandemly linked heavy chain variable regions from two *Llama*-derived single-domain antibodies N83G3.

SEQ ID NO: 15 shows the amino acid sequence of the tandemly linked heavy chain variable regions from two *Llama*-derived single-domain antibodies N83G3-h5.

SEQ ID NO: 16 shows the amino acid sequence of the anti-human CD3 single-chain antibody IMCR.

SEQ ID NO: 17 shows the amino acid sequence of the human (*homo sapiens*) CD3E extracellular region (hCD3E).

SEQ ID NO: 18 shows the amino acid sequence of the human (*homo sapiens*) CD3D extracellular region (hCD3D).

SEQ ID NO: 19 shows the amino acid sequence of the antigen peptide PRAME₃₀₁₋₃₀₉ derived from human (*homo sapiens*).

SEQ ID NO: 20 shows the amino acid sequence of the disulfide-trapped single-chain trimer complex (MIP-P3) of the antigen peptide PRAME₃₀₁₋₃₀₉ and HLA-A24.

SEQ ID NO: 21 shows the amino acid sequence of the His tag.

SEQ ID NO: 22 shows the amino acid sequence of the Fc segment (mFc) of the mouse (*mus musculus*) IgG2a antibody.

SEQ ID NO: 23 shows the amino acid sequence of the human (*homo sapiens*) IgG1 subtype antibody heavy chain constant region.

SEQ ID NO: 24 shows the amino acid sequence of the human IgG1 subtype antibody heavy chain constant region mutant IgG1H.

SEQ ID NO: 25 shows the amino acid sequence of the human IgG1 subtype antibody heavy chain constant region mutant IgG1K.

SEQ ID NO: 26 shows the amino acid sequence of the human IgG1 subtype antibody heavy chain constant region mutant IgG1m3-H.

SEQ ID NO: 27 shows the amino acid sequence of the human IgG1 subtype antibody heavy chain constant region mutant IgG1m3-K.

SEQ ID NO: 28 shows the amino acid sequence of the human IgG1 subtype antibody heavy chain constant region mutant IgG1m3-H1n1.

SEQ ID NO: 29 shows the amino acid sequence of the human IgG1 subtype antibody heavy chain constant region mutant IgG1m3-Kn1.

SEQ ID NO: 30 shows the amino acid sequence of the human (*homo sapiens*) λ isotype light chain constant region.

SEQ ID NO: 31 shows the amino acid sequence of the human (homo sapiens) κ isotype light chain constant region.

SEQ ID NO: 32 shows the amino acid sequence of the Fc segment (hFc) of the human (*homo sapiens*) IgG1 antibody.

SEQ ID NO: 33 shows the amino acid sequence of the human IgG1 subtype antibody Fc segment mutant IgG1m3-FcH1n1.

SEQ ID NO: 34 shows the amino acid sequence of the human IgG1 subtype antibody Fc segment mutant IgG1m3-FcKn1

SEQ ID NO: 35 shows the amino acid sequence of 2N83G3-G1m3-FcH1n1 in the bispecific antibody 2N83G3×IMCR.

SEQ ID NO: 36 shows the amino acid sequence of 2N83G3-h5-G1m3-FcH1n1 in the bispecific antibody 2N83G3-h5×IMCR.

SEQ ID NO: 37 shows the amino acid sequence of arm IMCR-scFv-G1m3-FcKn1 in the bispecific antibody 2N83G3×IMCR or 2N83G3-h5×IMCR.

SEQ ID NO: 38 shows the amino acid sequence of PRAME derived from human (*homo sapiens*).

SEQ ID NO: 39 shows the amino acid sequence of XCR1 derived from human (*homo sapiens*).

SEQ ID NO: 40 shows the amino acid sequence of KIFBP derived from human (*homo sapiens*).

SEQ ID NO: 41 shows the amino acid sequence of CPVL derived from human (*homo sapiens*).

SEQ ID NO: 42 shows the amino acid sequence of PLD1 derived from human (*homo sapiens*).

SEQ ID NO: 43 shows the amino acid sequence of HLA-A2402.

SEQ ID NO: 44 shows the amino acid sequence of the peptide segment 1 similar to PRAME₃₀₁₋₃₀₉ derived from human (*homo sapiens*).

SEQ ID NO: 45 shows the amino acid sequence of the peptide segment 2 similar to PRAME₃₀₁₋₃₀₉ derived from human (homo sapiens).

SEQ ID NO: 46 shows the amino acid sequence of the peptide segment 3 similar to PRAME₃₀₁₋₃₀₉ derived from human (*homo sapiens*).

SEQ ID NO: 47 shows the amino acid sequence of the peptide segment 4 similar to PRAME₃₀₁₋₃₀₉ derived from human (*homo sapiens*).

SEQ ID NO: 48 shows the amino acid sequence of the peptide segment 5 similar to PRAME₃₀₁₋₃₀₉ derived from human (*homo sapiens*).

SEQ ID NO: 49 shows the amino acid sequence of the peptide segment 6 similar to PRAME₃₀₁₋₃₀₉ derived from human (*homo sapiens*).

SEQ ID NO: 50 shows the amino acid sequence of the peptide segment 7 similar to PRAME₃₀₁₋₃₀₉ derived from human (*homo sapiens*).

SEQ ID NO: 51 shows the amino acid sequence of the peptide segment 8 similar to PRAME₃₀₁₋₃₀₉ derived from human (*homo sapiens*).

SEQ ID NO: 52 shows the amino acid sequence of the peptide segment 9 similar to PRAME₃₀₁₋₃₀₉ derived from human (*homo sapiens*).

SEQ ID NO: 53 shows the amino acid sequence of the peptide segment 10 similar to PRAME₃₀₁₋₃₀₉ derived from human (*homo sapiens*).

SEQ ID NO: 54 shows the amino acid sequence of the peptide segment 11 similar to PRAME₃₀₁₋₃₀₉ derived from human (*homo sapiens*).

SEQ ID NO: 55 shows the amino acid sequence of the peptide segment 12 similar to PRAME₃₀₁₋₃₀₉ derived from human (*homo sapiens*).

SEQ ID NO: 56 shows the amino acid sequence of the peptide segment 13 similar to PRAME₃₀₁₋₃₀₉ derived from human (*homo sapiens*).

SEQ ID NO: 57 shows the amino acid sequence of the peptide segment 14 similar to PRAME₃₀₁₋₃₀₉ derived from human (*homo sapiens*).

SEQ ID NO: 58 shows the amino acid sequence of the peptide segment 15 similar to PRAME₃₀₁₋₃₀₉ derived from human (*homo sapiens*).

SEQ ID NO: 59 shows the amino acid sequence of the peptide segment 16 similar to PRAME₃₀₁₋₃₀₉ derived from human (*homo sapiens*).

SEQ ID NO: 60 shows the amino acid sequence of the peptide segment 17 similar to PRAME₃₀₁₋₃₀₉ derived from human (*homo sapiens*).

SEQ ID NO: 61 shows the amino acid sequence of the peptide segment 18 similar to PRAME₃₀₁₋₃₀₉ derived from human (*homo sapiens*).

SEQ ID NO: 62 shows the amino acid sequence of the peptide segment 19 similar to PRAME₃₀₁₋₃₀₉ derived from human (*homo sapiens*).

SEQ ID NO: 63 shows the amino acid sequence of the peptide segment 20 similar to PRAME₃₀₁₋₃₀₉ derived from human (*homo sapiens*).

SEQ ID NO: 64 shows the amino acid sequence of the peptide segment 21 similar to PRAME₃₀₁₋₃₀₉ derived from human (*homo sapiens*).

SEQ ID NO: 65 shows the amino acid sequence of the peptide segment 22 similar to PRAME₃₀₁₋₃₀₉ derived from human (*homo sapiens*).

SEQ ID NO: 66 shows the amino acid sequence of the peptide segment 23 similar to PRAME₃₀₁₋₃₀₉ derived from human (*homo sapiens*).

SEQ ID NO: 67 shows the amino acid sequence of the peptide segment 24 similar to PRAME₃₀₁₋₃₀₉ derived from human (*homo sapiens*).

SEQ ID NO: 68 shows the amino acid sequence of the peptide segment 25 similar to PRAME₃₀₁₋₃₀₉ derived from human (*homo sapiens*).

SEQ ID NO: 69 shows the amino acid sequence of the peptide segment 26 similar to PRAME₃₀₁₋₃₀₉ derived from human (*homo sapiens*).

SEQ ID NO: 70 shows the amino acid sequence of the peptide segment 27 similar to PRAME₃₀₁₋₃₀₉ derived from human (*homo sapiens*).

SEQ ID NO: 71 shows the amino acid sequence of the peptide segment 28 similar to PRAME₃₀₁₋₃₀₉ derived from human (*homo sapiens*).

SEQ ID NO: 72 shows the amino acid sequence of the peptide segment 29 similar to PRAME₃₀₁₋₃₀₉ derived from human (*homo sapiens*).

SEQ ID NO: 73 shows the amino acid sequence of the peptide segment 30 similar to PRAME₃₀₁₋₃₀₉ derived from human (*homo sapiens*).

SEQ ID NO: 74 shows the amino acid sequence of the peptide segment 31 similar to PRAME₃₀₁₋₃₀₉ derived from human (*homo sapiens*).

SEQ ID NO: 75 shows the amino acid sequence of the peptide segment 32 similar to PRAME₃₀₁₋₃₀₉ derived from human (*homo sapiens*).

SEQ ID NO: 76 shows the amino acid sequence of the peptide segment 33 similar to PRAME₃₀₁₋₃₀₉ derived from human (*homo sapiens*).

SEQ ID NO: 77 shows the amino acid sequence of the peptide segment 34 similar to PRAME₃₀₁₋₃₀₉ derived from human (*homo sapiens*).

SEQ ID NO: 78 shows the amino acid sequence of the peptide segment 35 similar to PRAME₃₀₁₋₃₀₉ derived from human (*homo sapiens*).

SEQ ID NO: 79 shows the amino acid sequence of the peptide segment 36 similar to PRAME₃₀₁₋₃₀₉ derived from human (*homo sapiens*).

SEQ ID NO: 80 shows the amino acid sequence of the peptide segment 37 similar to PRAME₃₀₁₋₃₀₉ derived from human (*homo sapiens*).

SEQ ID NO: 81 shows the amino acid sequence of the peptide segment 38 similar to PRAME₃₀₁₋₃₀₉ derived from human (*homo sapiens*).

SEQ ID NO: 82 shows the amino acid sequence of the peptide segment 39 similar to PRAME₃₀₁₋₃₀₉ derived from human (*homo sapiens*).

SEQ ID NO: 83 shows the amino acid sequence of the peptide segment 40 similar to PRAME₃₀₁₋₃₀₉ derived from human (*homo sapiens*).

SEQ ID NO: 84 shows the amino acid sequence of the peptide segment 41 similar to PRAME₃₀₁₋₃₀₉ derived from human (*homo sapiens*).

SEQ ID NO: 85 shows the amino acid sequence of the peptide segment 42 similar to PRAME₃₀₁₋₃₀₉ derived from human (*homo sapiens*).

SEQ ID NO: 86 shows the amino acid sequence of the peptide segment 43 similar to PRAME₃₀₁₋₃₀₉ derived from human (*homo sapiens*).

SEQ ID NO: 87 shows the amino acid sequence of the peptide segment 44 similar to PRAME₃₀₁₋₃₀₉ derived from human (*homo sapiens*).

SEQ ID NO: 88 shows the amino acid sequence of the peptide segment 45 similar to PRAME₃₀₁₋₃₀₉ derived from human (*homo sapiens*).

SEQ ID NO: 89 shows the amino acid sequence of the peptide segment 46 similar to PRAME₃₀₁₋₃₀₉ derived from human (*homo sapiens*).

SEQ ID NO: 90 shows the amino acid sequence of the peptide segment 47 similar to PRAME₃₀₁₋₃₀₉ derived from human (*homo sapiens*).

SEQ ID NO: 91 shows the amino acid sequence of the peptide segment 48 similar to PRAME₃₀₁₋₃₀₉ derived from human (*homo sapiens*).

SEQ ID NO: 92 shows the amino acid sequence of the peptide segment 49 similar to PRAME₃₀₁₋₃₀₉ derived from human (*homo sapiens*).

SEQ ID NO: 93 shows the amino acid sequence of the peptide segment 50 similar to PRAME₃₀₁₋₃₀₉ derived from human (*homo sapiens*).

SEQ ID NO: 94 shows the amino acid sequence of the linker.

SEQ ID NO: 95 shows the nucleotide sequence of the primer PCal-CH2R.

SEQ ID NO: 96 shows the amino acid sequence of the negative control antibody DP47VH.

SEQ ID NO: 97 shows the amino acid sequence of the negative control antibody DP47VK.

SEQ ID NO: 98 shows the amino acid sequence of the irrelevant peptide.

### DETAILED DESCRIPTION OF THE INVENTION

The inventors of the present application prepared a bispecific antibody (e.g., TCRm bispecific antibodies) by genetically engineering a first antigen-binding fragment that binds to an HLA-A24/PRAME complex and a second antigen-binding fragment that binds to an activated T cell antigen (e.g., a CD3 molecule). The bispecific antibody binds to the complex of PRAME₃₀₁₋₃₀₉ (LYVDSLFFL) and HLA-A24 presented on the surface of a target cell (e.g., tumor cells) via the first antigen-binding fragment that binds to the HLA-A24/PRAME complex, and the second antigen-binding fragment binds to the activated T cell antigen (e.g., a CD3 molecule), thereby establishing an interaction between the target cell and the T cell. This interaction causes cytotoxic T-cell activation and lysis of the target cell (e.g., a tumor cell) in a histocompatibility complex (MHC)-dependent manner, achieving the purpose of treating a disease (e.g., a tumor). In various aspects, the present application provides a novel bispecific antibody comprising a first antigen-binding fragment that binds to an HLA-A24/PRAME complex and a second antigen-binding fragment that binds to an activated T cell antigen (e.g., a CD3 molecule), a single-domain antibody that binds to an HLA-A24/PRAME complex, a nucleic acid molecule encoding the bispecific antibody or the single-domain antibody, a vector comprising the nucleic acid molecule, a host cell comprising the nucleic acid molecule or the vector, a method for preparing and purifying the bispecific antibody or the single-domain antibody, and medical and biological uses of the bispecific antibody or the single-domain antibody. According to the sequence of the bispecific antibody or the single-domain antibody provided in the present application, the bispecific antibody or the single-domain antibody that binds to the HLA-A24/PRAME complex can be constructed for use as a medicament in clinical for preventing or treating a tumor.

Unless otherwise indicated, the inventions of the present application can be implemented using conventional molecular biology, microbiology, cell biology, biochemistry, and immunological techniques in the art.

Unless otherwise indicated, the terms used in the present application have the meanings commonly understood by those skilled in the art.

### Definition

As used herein, the term "HLA-A24/PRAME complex" refers to a complex of an HLA-A24 molecule and PRAME. In a specific embodiment of the present application, "HLA-A24/PRAME complex" refers to an HLA-A24/PRAME₃₀₁₋₃₀₉ complex of an HLA-A24 molecule and the amino acids at positions 301-309 of PRAME (PRAME₃₀₁₋₃₀₉) as set forth in SEQ ID NO: 38.

As used herein, the term "activated T cell antigen" refers to an antigenic determinant expressed on the surface of T lymphocytes, in particular cytotoxic T lymphocytes, which is capable of inducing T cell activation upon interaction with an antigen-binding molecule. In particular, the interaction between an antigen binding molecule and an activated T cell antigen may induce T cell activation by triggering a signal transduction cascade through T cell receptor complexes. In a particular aspect, the activated T cell antigen is a CD3 molecule.

As used herein, the term "antibody" refers to an immunoglobulin molecule capable of specifically binding to a target via at least one antigen recognition site located in its variable region. Targets include, but are not limited to, carbohydrates, polynucleotides, lipids, polypeptides, and the like. As used herein, "antibody" includes not only an intact (i.e., full-length) antibody, but also a binding fragment thereof (e.g., Fab, Fab', F(ab')₂, or Fv), a variant thereof, a fusion protein comprising an antibody moiety, a humanized antibody, a chimeric antibody, a bispecific antibody, a linear antibody, a single-chain antibody, a single-domain antibody, a multi-specific antibody (e.g., a bispecific antibody), and any other modified configurations of an immunoglobulin molecule comprising a desired specific antigen recognition site, including a glycosylated antibody variant, an amino acid sequence variant of an antibody, and a covalently modified antibody.

Typically, an intact or full-length antibody comprises two heavy chains and two light chains. Each heavy chain contains the heavy chain variable region (VH) and the first, second and third constant regions (CH1, CH2 and CH3). Each light chain contains the light chain variable region (VL) and the constant region (CL). A full-length antibody may belong to any immunoglobulin class, such as IgD, IgE, IgG, IgA, or IgM (or any of the above subclass), but it is not restricted to any particular type. Immunoglobulins are categorized based on the amino acid sequences of their heavy chain constant regions. Generally, there are five primary classes, i.e., IgA, IgD, IgE, IgG, and IgM, some of which are further divided into subclasses (subtypes), such as IgG1, IgG2, IgG3, IgG4, IgA1, and IgA2. The Heavy chain constant regions corresponding to these individual immunoglobulin classes are referred to as α, δ, ε, γ, and µ, respectively. Subunit structures and three-dimensional conformations of different types of immunoglobulins are well characterized.

As used herein, the term "bispecific antibody" refers to an antibody capable of simultaneously binding two distinct antigen epitopes. The two antigen epitopes can be on different antigens or on the same antigen. Bispecific antibodies can have a variety of structural configurations. For example, a bispecific antibody may consist of two Fc fragments and two binding moieties fused thereto, respectively (similar to a native antibody, except that the two arms bind to different antigen targets or epitopes), and the antigen-binding moieties can be a single-domain antibody, a single-chain variable fragment (scFv), or an Fab fragment. When targeting epitopes of given two antigens, two different binding moieties of the bispecific antibody each bind to the N-terminus of one Fc fragment, and the antigen-binding moiety of the two arms can be in one of four configurations: single-domain antibody + Fab fragment, single-domain antibody + scFv, scFv + single-domain antibody, and Fab fragment + single-domain antibody. The Fc fragment may comprise one or more mutations capable of ensuring heavy chain heteropolymerization, and the KIH technique (knob-into-hole) is a strategy to address this heavy chain heteropolymerization. Generally, the KIH technique refers to modification of the amino acid sequence of the CH3 region to form a structure that facilitates pairing of heterologous hemiantibodies to each other, which may construct a bispecific antibody while maintaining a normal antibody structure as much as possible. For guidance on the KIH technique, see, for example, "An efficient route to human bispecific IgG", A. Margaret Merchant et al., Nature Biotechnology, Volume 16, 1998^{[14]}, which is incorporated herein by reference in its entirety.

As used herein, the term "binding moiety" or "binding fragment" is used interchangeably to refer to the portion or region of an intact antibody molecule responsible for binding to an antigen. The antigen-binding domain may comprise a heavy chain variable region (VH), a light chain variable region (VL), or both. Each of the VH and VL typically contains three complementarity determining regions (CDR1, CDR2, and CDR3).

It is well known to those skilled in the art that complementarity determining regions (CDRs, typically including CDR1, CDR2 and CDR3) are the areas of a variable region having the greatest impact on the affinity and specificity of the antibody. The CDR sequence of the VH or VL has two common schemes, i.e., the Kabat numbering scheme and the Chothia numbering scheme (See, e.g., Kabat, "Sequences of Proteins of Immunological Interest", National Institutes of Health, Bethesda, Md. (1991) ^{[15]}; Al-Lazikani et al., J. Mol. Biol. 273:927-948 (1997) ^{[16]}; and Martin et al., Proc. Natl. Acad. Sci. USA. 86: 9268-9272 (1989)) ^{[17]}. For a given antibody variable region sequence, the CDR sequences in the VH and VL can be determined according to the Kabat or Chothia numbering scheme. In an embodiment of the present application, the CDR sequences are defined according to the Kabat numbering scheme.

For a given antibody variable region sequence, the CDR region sequences can be determined in various ways, for example, using the online software Abysis (http://www.abysis.org/).

Examples of an antigen-binding fragment for a typical antibody include, but are not limited to: (1) an Fab fragment, which is a monovalent fragment consisting of a VL-CL chain and a VH-CH1 chain; (2) an F(ab')₂ fragment, which is a divalent fragment consisting of two Fab' fragments linked via a disulfide bridge in the hinge region (i.e., a dimer of Fab'); (3) an Fv fragment consisting of a VL domain and a VH domain from a single arm of an antibody; (4) a single-chain Fv (scFv), which is a single polypeptide chain consisting of a VH domain and a VL domain via a peptide linker; (5) (scFv)₂, which comprises two VH domains linked via a peptide linker and two VL domains that associate with the two VH domains via disulfide bridges; and (6) a single-domain antibody form.

In bispecific antibody construction, a "binding moiety" includes, but is not limited to, a single-domain antibody form, an Fab fragment form, and/or a single-chain variable fragment (scFv) form.

As used herein, the term "single-chain variable fragment (scFv)" refers to a single-chain antibody generally constructed using genetic engineering techniques, comprising a polypeptide chain that includes both a heavy chain variable region (VH) and a light chain variable region (VL). A flexible linker is typically incorporated between the heavy chain variable region and the light chain variable region to enable proper folding of these regions into a correct conformation capable of binding to an antigen.

As used herein, the term "Fab (fragment antigen binding) fragment", "Fab moiety" or a similar term refers to an antibody fragment capable of binding to an antigen that is produced after papain digestion of an intact antibody comprising an intact light chain (VL-CL), and a heavy chain variable region and a CH1 fragment (VH-CH1).

As used herein, the term "single-domain antibody" refers to the variable domain of a naturally occurring heavy chain antibody that lacks light chains, comprising a heavy chain variable region (VHH) and conventional CH2 and CH3 regions (e.g., one or two groups (a heavy chain variable region (VHH) and conventional CH2 and CH3 regions)). The separately cloned and expressed VHH structure maintains structural stability comparable to its parent heavy chain antibody and retains antigen-binding activity, representing the smallest known antigen-binding unit. Single-domain antibodies are also known as nanobodies (Nb).

As used herein, the terms "Fc fragment", "Fc domain" and "Fc moiety" are used interchangeably to refer to the portion of an antibody heavy chain constant region that comprises the hinge region, CH2 domain and CH3 domain, as defined according to the EU numbering scheme for human IgG1 antibodies.

The term "specific binding" as used herein refers to a non-random binding reaction between two molecules, e.g., the binding of an antibody to an antigen epitope.

The term "tumor" as used herein refers to a neoplasm or solid lesion formed by abnormal cell growth. The tumor may be benign, premalignant or malignant.

As used herein, the term "malignant tumor" refers to or describes a pathological condition in mammals, typically characterized by unregulated cell growth. Exemplary malignant tumors include cancers, solid tumors, melanomas, sarcomas, hematological malignancies, germinomas, and blastomas. Further specific examples of malignant tumors include: melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, endometrial/uterine cancer, salivary adenocarcinoma, vulvar cancer, thyroid cancer, anal cancer, penile cancer, multiple myeloma, B-cell lymphoma, brain cancer, and associated metastases.

As used herein, the term "hematological tumor" refers to a tumor arising from uncontrolled growth of abnormal cells, which are in most cases derived from the bone marrow where blood cells are produced. Exemplary hematological tumors include various types of leukemia, multiple myeloma, and malignant lymphoma. Further specific examples of hematological tumors include acute lymphoblastic leukemia (ALL), chronic lymphocytic leukemia (CLL), acute myeloid leukemia (AML), chronic myeloid leukemia (CML), acute granulocytic leukemia, chronic granulocytic leukemia, hairy cell leukemia (HCL), T-cell prolymphocytic leukemia, large granule lymphocytic leukemia, juvenile myelomonocytic leukemia, B-cell prolymphocytic leukemia, Burkitt leukemia, adult T-cell leukemia, non-Hodgkin lymphoma, B-cell lymphoma, small lymphocytic lymphoma, lymphoplasmacytic lymphoma, primary macroglobulinemia (Waldenström macroglobulinemia), splenic marginal zone lymphoma, plasmacytoma, extranodal marginal zone B-cell lymphoma, MALT lymphoma, nodal marginal zone B-cell lymphoma (NMZL), follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma, B-cell chronic lymphocytic lymphoma, classical Hodgkin lymphoma, nodular lymphocyte-predominant Hodgkin lymphoma, adult T-cell lymphoma, extranodal NK/T-cell lymphoma (nasal type), Enteropathy-associated T-cell lymphoma, hepatosplenic T-cell lymphoma, NK-cell lymphoblastic lymphoma, mycosis fungoides, Sézary syndrome, primary cutaneous CD30+ T-cell lymphoproliferative disorder, primary cutaneous anaplastic large-cell lymphoma, lymphomatoid papulosis, angioimmunoblastic T-cell lymphoma, peripheral T-cell lymphoma not otherwise specified, and anaplastic large-cell lymphoma.

As used herein, the term "solid tumor" refers to an abnormal mass of tissue that can be detected by clinical examinations such as X-ray, CT scan, B-ultrasound, or palpation. Clinically diagnosed solid tumors are classified into two categories: malignant and benign. Malignant solid tumors include melanoma (including metastatic melanoma), head and neck cancer (e.g., head and neck squamous cell carcinoma), esophageal cancer (e.g., esophageal squamous cell carcinoma), urothelial cancer, oral cancer (e.g., oral squamous cell carcinoma), sarcomas (e.g., synovial sarcoma), liver cancer, lung cancer, bladder cancer, ovarian cancer, colorectal cancer, breast cancer; pediatric Hodgkin lymphoma including lymphocyte-predominant type, nodular sclerosis type, mixed cellularity type, and lymphocyte-depleted type; pediatric non-Hodgkin lymphoma including lymphoblastic lymphoma, small non-cleaved cell lymphoma (Burkitt/non-Burkitt lymphoma), diffuse large B-cell lymphoma, anaplastic large cell lymphoma, and the like; pediatric renal tumors including nephroblastoma (Wilms tumor), renal clear cell carcinoma, renal rhabdoid tumor, renal clear cell sarcoma, renal primitive neuroectodermal tumor, and the like; pediatric neuroblastic tumors including neuroblastoma, ganglioneuroblastoma, and ganglioneuroma; pediatric extracranial germinoma including mature teratoma, immature teratoma, endodermal sinus tumor (yolk sac tumor), seminoma, dysgerminoma, chorionic epithelioma, embryonic carcinoma, and the like; osteosarcoma and chondrosarcoma; pediatric rhabdomyosarcoma including embryonic type, acinar type, polymorphic type, and the like; soft tissue sarcomas including fibrosarcoma, malignant fibrous histiocytoma, liposarcoma, leiomyosarcoma, angiosarcoma, lymphangiosarcoma, malignant schwannoma, acinar soft tissue sarcoma, epithelioid sarcoma, clear cell sarcoma, malignant melanoma, synovial sarcoma, fibroproliferative small round cell tumor, and the like; Ewing sarcoma family of tumors including Ewing sarcoma and primary neuroectodermal tumor; pediatric liver tumors including hepatoblastoma (embryonal type, fetal type, and undifferentiated type), and hepatocellular carcinoma; retinoblastoma; and other tumors including medulloblastoma (posterior fossa), nasopharyngeal carcinoma, papillary thyroid carcinoma, thymoma, pulmonary blastoma, pancreatoblastoma, pancreatic islet cell tumor, ileocecal carcinoid, mesothelioma, and the like. Benign solid tumors include lymphangiomas, hemangiomas, thyroglossal duct cysts, and the like.

In a first aspect, the present application provides a bispecific antibody comprising a first antigen binding fragment that binds to an HLA-A24/PRAME complex and a second antigen binding fragment that binds to an activated T cell antigen.

In some embodiments of the first aspect, the bispecific antibody is capable of mediating activation of T cells in response to HLA-A24⁺/PRAME⁺ tumor cells, and/or the bispecific antibody is capable of mediating the killing of HLA-A24⁺/PRAME⁺ tumor cells by PBMCs.

In some embodiments of the first aspect, the bispecific antibody is capable of mediating activation of Jurkat-Dual cells by HLA-A24⁺/PRAME⁺ tumor cells.

In some embodiments of the first aspect, the first antigen-binding fragment binds to the HLA-A24/PRAME complex at an epitope comprising one or more residues at positions 301-309 of PRAME as set forth in SEQ ID NO: 38. In some embodiments, the amino acid sequence at positions 301-309 of PRAME as set forth in SEQ ID NO: 38 is LYVDSLFFL (SEQ ID NO: 19).

In some embodiments of the first aspect, the first antigen-binding fragment binds to the HLA-A24/PRAME complex at an epitope comprising at least one of residues at positions 304, 305, 306, 307, 308, and 309 of PRAME as set forth in SEQ ID NO: 38.

In some embodiments of the first aspect, the first antigen-binding fragment binds to the HLA-A24/PRAME complex at an epitope comprising residues at positions 304, 305, 306, 307, 308, and 309 of PRAME as set forth in SEQ ID NO: 38. In some embodiments, residues at positions 304, 305, 306, 307, 308, and 309 of PRAME as set forth in SEQ ID NO: 38 correspond to amino acid residues at positions 4, 5, 6, 7, 8, and 9 of PRAME₃₀₁₋₃₀₉ (LYVDSLFFL) polypeptide as set forth in SEQ ID NO: 19.

In some embodiments of the first aspect, the activated T cell antigen is a CD3 molecule.

In some embodiments of the first aspect, the first antigen-binding fragment comprises HCDR1 as set forth in SEQ ID NO: 1, HCDR2 as set forth in SEQ ID NO: 2, and HCDR3 as set forth in SEQ ID NO: 3; wherein the amino acid sequence of HCDR is defined according to Kabat numbering scheme.

In some embodiments of the first aspect, the first antigen-binding fragment is in the form of a single-domain antibody.

In some embodiments of the first aspect, the first antigen-binding fragment comprises a monovalent or multivalent (e.g., monovalent, bivalent, trivalent, quadrivalent, pentavalent, or hexavalent) single-domain antibody that binds to the HLA-A24/PRAME complex.

In some embodiments of the first aspect, the first antigen-binding fragment comprises a divalent single-domain antibody that binds to the HLA-A24/PRAME complex. In some embodiments, the first antigen-binding fragment comprises heavy chain variable regions of two monovalent single-domain antibodies that bind to the HLA-A24/PRAME complex.

In some embodiments of the first aspect, the first antigen-binding fragment comprises directly fused heavy chain variable regions of two monovalent single-domain antibodies that bind to the HLA-A24/PRAME complex.

In some embodiments of the first aspect, the first antigen-binding fragment comprises heavy chain variable regions of two monovalent single-domain antibodies that bind to the HLA-A24/PRAME complex linked via a linker. In some embodiments, the linker is a GS-type flexible peptide linker. In some embodiments, the linker is (G4S)n, (SG4)n, or G4(SG4)n, where n is an integer from 1 to 10, e.g., 1, 2, 3, 4, 5, 6, 7, 8, 9, 10, or an integer or range of integers between any two of the foregoing values. In some embodiments, the linker is (G4S)n, (SG4)n, or G4(SG4)n, wherein n is an integer from 2 to 4, such as 2, 3, or 4. In some specific embodiments, the linker is GGGGSGGGGSGGGGS (SEQ ID NO: 94).

In some embodiments of the first aspect, the second antigen-binding fragment comprises HCDR1 as set forth in SEQ ID NO: 6, HCDR2 as set forth in SEQ ID NO: 7, HCDR3 as set forth in SEQ ID NO: 8, LCDR1 as set forth in SEQ ID NO: 9, LCDR2 as set forth in SEQ ID NO: 10, and LCDR3 as set forth in SEQ ID NO: 11; wherein the amino acid sequence of HCDR is defined according to Kabat numbering scheme.

In some embodiments of the first aspect, the second antigen-binding fragment is in the form of a single-chain variable fragment (scFv) or an Fab fragment.

In some embodiments of the first aspect, the second antigen-binding fragment is in the form of an scFv.

In some embodiments of the first aspect, the first antigen-binding fragment comprises the amino acid sequence as set forth in SEQ ID NO: 4.

In some embodiments of the first aspect, the first antigen-binding fragment comprises the amino acid sequence as set forth in SEQ ID NO: 5.

In some embodiments of the first aspect, the first antigen-binding fragment comprises the amino acid sequence as set forth in SEQ ID NO: 14.

In some embodiments of the first aspect, the first antigen-binding fragment comprises the amino acid sequence as set forth in SEQ ID NO: 15.

In some embodiments of the first aspect, the amino acid sequence as set forth in SEQ ID NO: 14 comprises two amino acid sequences as set forth in SEQ ID NO: 4.

In some embodiments of the first aspect, the amino acid sequence as set forth in SEQ ID NO: 15 comprises two amino acid sequences as set forth in SEQ ID NO: 5.

In some embodiments of the first aspect, the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises the amino acid sequence as set forth in SEQ ID NO: 4; and the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises the amino acid sequence as set forth in SEQ ID NO: 5; and the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises the amino acid sequence as set forth in SEQ ID NO: 14; and the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises the amino acid sequence as set forth in SEQ ID NO: 15; and the second antigen-binding fragment comprises a heavy chain variable region having the amino acid sequence as set forth in SEQ ID NO: 12 and a light chain variable region having the amino acid sequence as set forth in SEQ ID NO: 13.

In some embodiments of the first aspect, the first antigen-binding fragment comprises an amino acid sequence differing from the amino acid sequence as set forth in SEQ ID NO: 4, 5, 14, or 15 by about 1, 2, 3, 4, 5, 6, 7, 8, 9, or 10 amino acid substitutions, deletions, and/or additions.

In some embodiments of the first aspect, the first antigen-binding fragment comprises an amino acid sequence having at least 90%, 91%, 92%, 93%, 94%, 95%, 96%, 97%, 98%, 99% or higher homology to the amino acid sequence as set forth in SEQ ID NO: 4, 5, 14, or 15.

In some embodiments of the first aspect, the amino acid sequence as set forth in SEQ ID NO: 4, 5, 14, or 15 can also be truncated at the C-terminus or N-terminus by about 1, 2, 3, 4, 5, 6, 7, 8, 9, 10, 11, 12, 13, 14, 15, 20, 25, or more amino acids while still retaining the similar function of the first antigen-binding fragment.

In some embodiments of the first aspect, the amino acid sequence as set forth in SEQ ID NO: 4, 5, 14 or 15 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the first antigen-binding fragment.

In some embodiments of the first aspect, the amino acid sequence as set forth in SEQ ID NO: 4, 5, 14, or 15 can also have 1, 2, 3, 4, 5, 6, 7, 8, 9, 10, 11, 12, 13, 14, 15, 20, 25 or more amino acids added or deleted at regions other than the C-terminus or N-terminus, provided that the altered amino acid sequence substantially retains similar function of the first antigen-binding fragment.

In some embodiments of the first aspect, the heavy chain variable region of the second antigen binding fragment comprises the amino acid sequence as set forth in SEQ ID NO: 12.

In some embodiments of the first aspect, the heavy chain variable region of the second antigen binding fragment comprises an amino acid sequence differing from the amino acid sequence as set forth in SEQ ID NO: 12 by about 1, 2, 3, 4, 5, 6, 7, 8, 9, or 10 amino acid substitutions, deletions, and/or additions.

In some embodiments of the first aspect, the heavy chain variable region of the second antigen binding fragment comprises an amino acid sequence having at least 90%, 91%, 92%, 93%, 94%, 95%, 96%, 97%, 98%, 99% or higher homology to the amino acid sequence as set forth in SEQ ID NO: 12.

In some embodiments of the first aspect, the amino acid sequence as set forth in SEQ ID NO: 12 can also be truncated at the C-terminus or N-terminus by about 1, 2, 3, 4, 5, 6, 7, 8, 9, 10, 11, 12, 13, 14, 15, 20, 25 or more amino acids while still retaining the similar function of the heavy chain variable region of the second antigen-binding fragment.

In some embodiments of the first aspect, the amino acid sequence as set forth in SEQ ID NO: 12 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the heavy chain variable region of the second antigen-binding fragment.

In some embodiments of the first aspect, the amino acid sequence as set forth in SEQ ID NO: 12 can also have 1, 2, 3, 4, 5, 6, 7, 8, 9, 10, 11, 12, 13, 14, 15, 20, 25 or more amino acids added or deleted at regions other than the C-terminus or the N-terminus, provided that the altered amino acid sequence substantially retains the similar function of the heavy chain variable region of the second antigen-binding fragment.

In some embodiments of the first aspect, the light chain variable region of the second antigen binding fragment comprises the amino acid sequence as set forth in SEQ ID NO: 13.

In some embodiments of the first aspect, the light chain variable region of the second antigen-binding fragment comprises an amino acid sequence differing from the amino acid sequence as set forth in SEQ ID NO: 13 by about 1, 2, 3, 4, 5, 6, 7, 8, 9, or 10 amino acid substitutions, deletions, and/or additions.

In some embodiments of the first aspect, the amino acid sequence of the light chain variable region of the second antigen binding fragment has at least 90%, 91%, 92%, 93%, 94%, 95%, 96%, 97%, 98%, 99% or higher homology to the amino acid sequence as set forth in SEQ ID NO: 13.

In some embodiments of the first aspect, the amino acid sequence as set forth in SEQ ID NO: 13 can also be truncated at the C-terminus or N-terminus by about 1, 2, 3, 4, 5, 6, 7, 8, 9, 10, 11, 12, 13, 14, 15, 20, 25 or more amino acids while still retaining the similar function of the light chain variable region of the second antigen-binding fragment.

In some embodiments of the first aspect, the amino acid sequence as set forth in SEQ ID NO: 13 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the light chain variable region of the second antigen-binding fragment.

In some embodiments of the first aspect, the amino acid sequence as set forth in SEQ ID NO: 13 can also have 1, 2, 3, 4, 5, 6, 7, 8, 9, 10, 11, 12, 13, 14, 15, 20, 25 or more amino acids added or deleted at regions other than the C-terminus or the N-terminus, provided that the altered amino acid sequence substantially retains the similar function of the light chain variable region of the second antigen-binding fragment.

In some embodiments of the first aspect, the first antigen binding fragment and the second antigen binding fragment are linked via an Fc fragment of an antibody heavy chain constant region comprising a first Fc fragment and a second Fc fragment, wherein the first Fc fragment comprises the amino acid C at position 354 and the amino acid W at position 366; or the amino acid C at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407; wherein the amino acid positions of the antibody constant region are determined according to EU numbering scheme.

In some embodiments of the first aspect, the first antigen binding fragment and the second antigen binding fragment are linked via an Fc fragment of an antibody heavy chain constant region comprising a first Fc fragment and a second Fc fragment, wherein the first Fc fragment comprises the amino acid C at position 354 and the amino acid W at position 366, and the second Fc fragment comprises the amino acid C at position 349, the amino acid S at position 366, the amino acid A at position 368, and the amino acid V at position 407; wherein the amino acid positions of the antibody constant region are determined according to EU numbering scheme.

In some embodiments of the first aspect, the first antigen binding fragment and the second antigen binding fragment are linked via an Fc fragment of an antibody heavy chain constant region comprising a first Fc fragment and a second Fc fragment, wherein the first Fc fragment and the second Fc fragment comprise the amino acid F at position 234, the amino acid E at position 235 and the amino acid S at position 331, respectively; wherein the amino acid positions of the antibody constant region are determined according to EU numbering.

In some embodiments of the first aspect, the first antigen binding fragment and the second antigen binding fragment are linked via an Fc fragment of an antibody heavy chain constant region comprising a first Fc fragment and a second Fc fragment, wherein one of the first Fc fragment and the second Fc fragment is linked to the first antigen binding fragment and the other of the first Fc fragment and the second Fc fragment is linked to the second antigen binding fragment.

In some embodiments of the first aspect, the first antigen-binding fragment (e.g., at the C-terminus) is linked to the N-terminus of the first Fc fragment (e.g., having the amino acid sequence as set forth in SEQ ID NO: 33).

In some embodiments of the first aspect, the second antigen-binding fragment (e.g., at the C-terminus) is linked to the N-terminus of the second Fc fragment (e.g., having the amino acid sequence as set forth in SEQ ID NO: 34).

In some embodiments of the first aspect, the Fc fragment of the antibody heavy chain constant region is an Fc fragment of the IgG1 subtype. In some embodiments, the first Fc fragment is an Fc fragment of the IgG1 subtype; and/or the second Fc fragment is an Fc fragment of the IgG1 subtype.

In some embodiments of the first aspect, the Fc fragment of the antibody heavy chain constant region is an Fc fragment of the IgG1m3 allotype. In some embodiments, the first Fc fragment is an Fc fragment of the IgG1m3 allotype; and/or the second Fc fragment is an Fc fragment of the IgG1m3 allotype.

In some embodiments of the first aspect, the bispecific antibody comprises a first arm that binds to the HLA-A24/PRAME complex and a second arm that binds to the activated T cell antigen, wherein the first arm comprises the amino acid sequence as set forth in SEQ ID NO: 35 or 36; and the second arm comprises the amino acid sequence as set forth in SEQ ID NO: 37.

In some embodiments of the first aspect, the first arm comprises the amino acid sequence as set forth in SEQ ID NO: 35 or 36.

In some embodiments of the first aspect, the first arm comprises an amino acid sequence differing from the amino acid sequence as set forth in SEQ ID NO: 35 or 36 by about 1, 2, 3, 4, 5, 6, 7, 8, 9, or 10 amino acid substitutions, deletions, and/or additions.

In some embodiments of the first aspect, the first arm comprises an amino acid sequence having at least 90%, 91%, 92%, 93%, 94%, 95%, 96%, 97%, 98%, 99% or higher homology to the amino acid sequence as set forth in SEQ ID NO: 35 or 36.

In some embodiments of the first aspect, the amino acid sequence as set forth in SEQ ID NO: 35 or 36 can also be truncated at the C-terminus or N-terminus by about 1, 2, 3, 4, 5, 6, 7, 8, 9, 10, 11, 12, 13, 14, 15, 20, 25 or more amino acids while still retaining the similar function of the first arm.

In some embodiments of the first aspect, the amino acid sequence as set forth in SEQ ID NO: 35 or 36 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the first arm.

In some embodiments of the first aspect, the amino acid sequence as set forth in SEQ ID NO: 35 or 36 can also have 1, 2, 3, 4, 5, 6, 7, 8, 9, 10, 11, 12, 13, 14, 15, 20, 25 or more amino acids added or deleted at regions other than the C-terminus or the N-terminus, provided that the altered amino acid sequence substantially retains the similar function of the first arm.

In some embodiments of the first aspect, the second arm comprises the amino acid sequence as set forth in SEQ ID NO: 37.

In some embodiments of the first aspect, the second arm comprises an amino acid sequence differing from the amino acid sequence as set forth in SEQ ID NO: 37 by about 1, 2, 3, 4, 5, 6, 7, 8, 9, or 10 amino acid substitutions, deletions, and/or additions.

In some embodiments of the first aspect, the second arm comprises an amino acid sequence having at least 90%, 91%, 92%, 93%, 94%, 95%, 96%, 97%, 98%, 99% or higher homology to the amino acid sequence as set forth in SEQ ID NO: 37.

In some embodiments of the first aspect, the amino acid sequence as set forth in SEQ ID NO: 37 can also be truncated at the C-terminus or N-terminus by about 1, 2, 3, 4, 5, 6, 7, 8, 9, 10, 11, 12, 13, 14, 15, 20, 25 or more amino acids while still retaining the similar function of the second arm.

In some embodiments of the first aspect, the amino acid sequence as set forth in SEQ ID NO: 37 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the second arm.

In some embodiments of the first aspect, the amino acid sequence as set forth in SEQ ID NO: 37 can also have 1, 2, 3, 4, 5, 6, 7, 8, 9, 10, 11, 12, 13, 14, 15, 20, 25 or more amino acids added or deleted at regions other than the C-terminus or the N-terminus, provided that the altered amino acid sequence substantially retains the similar function of the second arm.

In a second aspect, the present application provides a single-domain antibody that binds to an HLA-A24/PRAME complex, comprising: HCDR1 as set forth in SEQ ID NO: 1, HCDR2 as set forth in SEQ ID NO: 2, and HCDR3 as set forth in SEQ ID NO: 3; wherein the amino acid sequences of HCDRs are defined according to Kabat numbering scheme.

In some embodiments of the second aspect, the single-domain antibody binds to the HLA-A24/PRAME complex at an epitope comprising one or more of residues at positions 301-309 of PRAME as set forth in SEQ ID NO: 38. In some embodiments, the amino acid sequence at positions 301-309 of PRAME as set forth in SEQ ID NO: 38 is LYVDSLFFL (SEQ ID NO: 19).

In some embodiments of the second aspect, the single-domain antibody binds to the HLA-A24/PRAME complex at an epitope comprising at least one of residues at positions 304, 305, 306, 307, 308, and 309 of PRAME as set forth in SEQ ID NO: 38.

In some embodiments of the second aspect, the single-domain antibody binds to the HLA-A24/PRAME complex at an epitope comprising residues at positions 304, 305, 306, 307, 308, and 309 of PRAME as set forth in SEQ ID NO: 38. In some embodiments, residues at positions 304, 305, 306, 307, 308, and 309 of PRAME as set forth in SEQ ID NO: 38 correspond to amino acid residues at positions 4, 5, 6, 7, 8, and 9 of PRAME₃₀₁₋₃₀₉ (LYVDSLFFL) polypeptide as set forth in SEQ ID NO: 19.

In some embodiments of the second aspect, the single-domain antibody is in the form of a monovalent or multivalent single-domain antibody that binds to the HLA-A24/PRAME complex.

In some embodiments of the second aspect, the single-domain antibody is in the form of a monovalent single-domain antibody that binds to the HLA-A24/PRAME complex.

In some embodiments of the second aspect, the single-domain antibody is in the form of a bivalent single-domain antibody that binds to the HLA-A24/PRAME complex.

In some embodiments of the second aspect, the single-domain antibody comprises heavy chain variable regions of two monovalent single-domain antibodies that bind to the HLA-A24/PRAME complex.

In some embodiments of the second aspect, the single-domain antibody comprises directly fused heavy chain variable regions of two monovalent single-domain antibodies that bind to the HLA-A24/PRAME complex.

In some embodiments of the second aspect, the single-domain antibody comprises heavy chain variable regions of two monovalent single-domain antibodies that bind to the HLA-A24/PRAME complex linked via a linker. In some embodiments, the linker is a GS-type flexible peptide linker. In some embodiments, the linker is (G4S)n, (SG4)n, or G4(SG4)n, where n is an integer from 1 to 10, e.g., 1, 2, 3, 4, 5, 6, 7, 8, 9, 10, or an integer or range of integers between any two of the foregoing values. In some embodiments, the linker is (G4S)n, (SG4)n, or G4(SG4)n, wherein n is an integer from 2 to 4, such as 2, 3, or 4. In some specific embodiments, the linker is GGGGSGGGGSGGGGS (SEQ ID NO: 94).

In some embodiments of the second aspect, the single-domain antibody comprises the amino acid sequence as set forth in SEQ ID NO: 4.

In some embodiments of the second aspect, the single-domain antibody comprises the amino acid sequence as set forth in SEQ ID NO: 5.

In some embodiments of the second aspect, the single-domain antibody comprises the amino acid sequence as set forth in SEQ ID NO: 14.

In some embodiments of the second aspect, the single-domain antibody comprises the amino acid sequence as set forth in SEQ ID NO: 15.

In some embodiments of the second aspect, the single-domain antibody comprises an amino acid sequence differing from the amino acid sequence as set forth in SEQ ID NO: 4, 5, 14, or 15 by about 1, 2, 3, 4, 5, 6, 7, 8, 9, or 10 amino acid substitutions, deletions, and/or additions.

In some embodiments of the second aspect, the single-domain antibody comprises an amino acid sequence having at least 90%, 91%, 92%, 93%, 94%, 95%, 96%, 97%, 98%, 99%, or higher homology to SEQ ID NO: 4, 5, 14, or 15.

In some embodiments of the second aspect, the amino acid sequence as set forth in SEQ ID NO: 4, 5, 14, or 15 can also be truncated at the C-terminus or N-terminus by about 1, 2, 3, 4, 5, 6, 7, 8, 9, 10, 11, 12, 13, 14, 15, 20, 25, or more amino acids while still retaining the similar function of the single-domain antibody.

In some embodiments of the second aspect, the amino acid sequence as set forth in SEQ ID NO: 4, 5, 14 or 15 can also have 1, 2, 3, 4, 5, 6, 7, 8, 9, 10, 11, 12, 13, 14, 15, 20, 25 or more amino acids added at the C-terminus or N-terminus, and the resulting amino acid sequence still retains the similar function of the single-domain antibody.

In some embodiments of the second aspect, the amino acid sequence as set forth in SEQ ID NO: 4, 5, 14 or 15 can also have 1, 2, 3, 4, 5, 6, 7, 8, 9, 10, 11, 12, 13, 14, 15, 20, 25 or more amino acids added or deleted at regions other than the C-terminus or the N-terminus, provided that the altered amino acid sequence substantially retains the similar function of the single-domain antibody.

In a third aspect, the application provides a nucleic acid molecule encoding the bispecific antibody of the first aspect or the single-domain antibody of the second aspect.

In some embodiments of the third aspect, the nucleic acid molecule can include a DNA molecule and an RNA molecule. The nucleic acid molecule can be single-stranded or doublestranded, and can be cDNA.

In some embodiments of the third aspect, the nucleic acid molecule is operably linked to a regulatory nucleotide sequence which can be recognized by a host cell transformed with a vector.

In a fourth aspect, the present application provides a pharmaceutical composition comprising the bispecific antibody of the first aspect or the single-domain antibody of the second aspect, and a pharmaceutically acceptable excipient, diluent or carrier.

In some embodiments of the fourth aspect, the pharmaceutical composition can be used to prevent or treat an HLA-A24/PRAME-positive tumor.

In some embodiments of the fourth aspect, the HLA-A24/PRAME positive tumor can be selected from the group consisting of melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).

In some embodiments of the fourth aspect, the pharmaceutical composition can further comprise one or more of the following substances: lubricants such as talc, magnesium stearate, and mineral oil; wetting agents; emulsifiers; suspending agents; preservatives such as benzoic acid, sorbic acid and calcium propionate; sweetening agents and/or flavoring agents, and the like.

In some embodiments of the fourth aspect, the pharmaceutical composition of the present application can be formulated as tablets, pills, powders, lozenges, elixirs, suspensions, emulsions, solutions, syrups, suppositories, capsules, or the like.

In some embodiments of the fourth aspect, the pharmaceutical composition of the present application can be delivered using any physiologically acceptable administration route including, but not limited to: oral, parenteral, nasal, rectal, intraperitoneal, intravascular, subcutaneous, transdermal, or inhalation administration.

In some embodiments of the fourth aspect, the pharmaceutical composition for therapeutic use can be formulated for storage in the form of a lyophilized formulation or an aqueous solution by mixing reagents having the desired purity with, as appropriate, pharmaceutically acceptable carriers, excipients, and the like.

In a fifth aspect, the present application provides use of the bispecific antibody of the first aspect, the single-domain antibody of the second aspect, or the pharmaceutical composition of the fourth aspect in the manufacture of a medicament for the prevention or treatment of an HLA-A24/PRAME-positive tumor.

In some embodiments of the fifth aspect, the HLA-A24/PRAME positive tumor is selected from the group consisting of melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).

In a sixth aspect, the present application provides a method for preventing or treating an HLA-A24/PRAME-positive tumor, comprising administering to a subject in need thereof the bispecific antibody of the first aspect, the single-domain antibody of the second aspect, or the pharmaceutical composition of the fourth aspect.

In some embodiments of the sixth aspect, the HLA-A24/PRAME positive tumor is selected from the group consisting of melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).

In a seventh aspect, the present application provides use of the bispecific antibody of the first aspect or the single-domain antibody of the second aspect in the manufacture of a product for detecting HLA-A24/PRAME positive cells.

In some embodiments of the seventh aspect, the product is a kit, a test strip, a test card, or a microfluidic device.

In some embodiments of the seventh aspect, the product may further comprise a detection label, such as colloidal gold, a chemiluminescent label, a fluorescent label, a nanoparticle label, and the like.

In some embodiments of the seventh aspect, the product may further comprise other reagents used in detection, such as an enzyme- or colloidal gold-labeled antigen or antibody, a substrate, a reference standard, a diluent, a wash solution, and the like.

In some embodiments of the seventh aspect, the product may further comprise a reagent for processing a biological sample for detection, and the biological sample can be blood.

In some embodiments of the seventh aspect, the product may further comprise instructions for use.

The present application also provides a vector comprising a nucleic acid molecule encoding the bispecific antibody of the first aspect or the single-domain antibody of the second aspect, and a host cell comprising the nucleic acid molecule or the vector. In other aspects, the application also provides a method of producing the bispecific antibody of the first aspect or the single-domain antibody of the second aspect. In some embodiments, the method of producing the bispecific antibody of the first aspect or the single-domain antibody of the second aspect comprises culturing the host cell in order to express the nucleic acid molecule. In some embodiments, the method of producing the bispecific antibody of the first aspect, or the single-domain antibody of the second aspect, further comprises recovering the bispecific antibody or the single-domain antibody from the host cell culture medium.

It is to be understood that the foregoing detailed description is intended only to enable those skilled in the art to have a clearer understanding of the present application and is not intended to be limiting in any way. Various modifications and variations may be made to the embodiments by those skilled in the art.

The following examples are for purposes of illustration only and are not intended to limit the scope of the present application.

### Examples

### Example 1: Preparation of Recombinant Proteins

A variety of different recombinant proteins, including the human CD3E extracellular domain (hCD3E, SEQ ID NO: 17) and the human CD3D extracellular domain (hCD3D, SEQ ID NO: 18), were necessary for preparing a bispecific antibody targeting HLA-A24/PRAME and CD3. Meanwhile, the following was prepared according to the reference ^{[18]}: the disulfide trap single-chain trimer (dtSCT) structure complex (MIP-P3, SEQ ID NO: 20) of PRAME antigen peptide LYVDSLFFL (SEQ ID NO: 19) and HLA-A24. These recombinant proteins all have many post-translation modifications (e.g., glycosylation or disulfide bonds), and thus it would be advantageous to use a mammal cell expression system to maintain their structure and function. To facilitate protein purification and monoclonal antibody functional identification, a His tag (His, SEQ ID NO: 21) or the Fc fragment of murine antibody IgG2a (mFc, SEQ ID NO: 22) was added to the C-terminus of the recombinant proteins. In the preparation of recombinant antibodies, the antibody heavy chain constant region can be the human IgG1 subtype (SEQ ID NO: 23) or its various variants, such as IgG1H (SEQ ID NO: 24), IgG1K (SEQ ID NO: 25), IgG1m3-H (SEQ ID NO: 26), IgG1m3-K (SEQ ID NO: 27), IgG1m3-H1n1 (SEQ ID NO: 28) or IgG1m3-Kn1 (SEQ ID NO: 29). The light chain constant region can be the human λ isotype (SEQ ID NO: 30) or the human κ isotype (SEQ ID NO: 31).

Genes including the His tag or mFc tag coding gene for the various recombinant proteins described above were designed and synthesized according to the amino acid sequences of the recombinant proteins of interest in the Uniprot database. The synthesized genes for the various recombinant proteins were cloned into suitable eukaryotic expression vectors (e.g., pcDNA3.1 from Invitrogen Inc.) using conventional molecular biology techniques, and the plasmids prepared for recombinant protein expression were then transfected into HEK293 cells (e.g., HEK293F from Invitrogen Inc.) using liposomes (e.g., 293fectin from Invitrogen Inc.) or other cationic transfection reagents (e.g., PEI). The cells were cultured in suspension under serum-free conditions for 3-4 days. The culture supernatant was then harvested by centrifugation or the like.

The recombinant proteins expressed as fusions with His tag were subjected to one-step purification from the culture supernatant using a metal chelate affinity chromatography column (e.g., HisTrap FF from GE Co.). The recombinant proteins expressed as fusions with mFc tag were subjected to one-step purification using a ProteinA/G affinity chromatography column (such as Mabselect SURE from GE Co.). The recombinant protein preservation buffer was then replaced with PBS (pH 7.0) or another suitable buffer using a desalting column (e.g., Hitrap desaulting from GE Co.). If necessary, the recombinant protein samples can be sterilized by filtration, and then stored in aliquots at -20°C.

### Example 2: Screening and Activity Evaluation of Anti-HLA-A24/PRAME Monoclonal Antibodies

### 2.1 Llama Immunization and Preparation of Single-domain Antibody Library

One healthy adult llama was selected, and blood was collected to obtain baseline serum before immunization. For the first immunization, 0.4 mg of MIP-P3-His fusion protein was emulsified with Freund's Complete Adjuvant, and then subcutaneously injected at multiple sites for immunization; For booster immunizations every two weeks, 0.4 mg of MIP-P3-His fusion protein was emulsified with Freund's Incomplete Adjuvant, and then subcutaneously injected at multiple sites., A total of five booster immunizations were conducted, and blood was collected before each immunization for antibody titer analysis. For the seventh immunization, 0.4 mg of MIP-P3-His fusion protein without adjuvant was taken as the antigen, and subcutaneously injected at multiple sites for high-dose booster immunization. 150 mL of peripheral blood was collected for lymphocyte separation after 3 days.

llama peripheral blood lymphocytes were separated from150 mL of peripheral blood using Camel Peripheral Blood Lymphocyte Isolation Kit (Solarbio, CAT#P5750). Total RNA of the lymphocytes was extracted using Total RNA Extraction Kit (TIANGEN BIOTECH (BEIJING) Co., Ltd., CAT#DP430). The extracted total RNA was used as a template to synthesize the llama heavy chain variable region using First-Strand cDNA Synthesis Kit (Thermo scientific, CAT#K1621). Gene specific primers were used as primers for reverse transcription, and the primer pair region was located in the CH2 domain of the antibody heavy chain constant region, with the specific sequence of PCal-CH2R as: TCCTTCCCCGTCAGCCAGTCCT (SEQ ID NO: 95). The synthesized cDNAs were immediately stored at -70°C for future use. Then, the cDNAs obtained by reverse transcription was used as a template to synthesize primers according to the reference ^{[19]}, and the llama VHH gene was amplified by nested PCR and isolated. Finally, the amplified VHH genes were cloned into vector pADSCFV-S (see, the refernce ^{[20]}) to construct a VHH library. The antibody library had a capacity of 2.0E8, with an accuracy of 66.67%.

### 2.2 Screening of Llama Immune Library

According to the reference (see Chinese Patent Application No. 201510097117.0 ^{[20]}), the phage library displaying llama single-domain antibodies constructed in Section 2.1 was screened by a solid phase screening strategy (see, Phage Display: A Practical Approach, Clackson, T. (USA) and Lowman, H. B. (USA) (ed.), translated by Lan Ma et al., Chemical Industry Press, May 2008.5 [19] for the experimental protocol) using the recombinant protein MIP-P3-mFc prepared in Example 1 as antigens. Three rounds of screening were performed by means of binding, elution, neutralization, infection and amplification. Finally, a TCRm antibody N83G3 (SEQ ID NO: 4) that specifically binds to MIP-P3-His was obtained.

By using conventional molecular biological methods, the nucleotide sequences encoding N83G3 heavy chain variable regions were respectively cloned into a eukaryotic expression vector (e.g., pcDNA3.1 from Invitrogen Inc.) fused with the nucleotide sequence encoding the Fc region (Fc, SEQ ID NO: 32) of the human antibody IgG1 to express N83G3-Fc recombinant proteins.

### 2.3 Affinity Analysis of Anti-HLA-A24/PRAME Antibody

The affinity of anti-HLA-A24/PRAME monoclonal antibodies was determined by the surface plasmon resonance technique using Biacore T200. Related reagents and consumables, such as the Amino Coupling Kit (BR-1000-50), the Human Antibody Capturing Kit (BR-1008-39), a S-series CM5 chip (14100530), and 10×HBS-EP (BR100669) at pH 7.4 were all purchased from GE healthcare. According to the kit instructions, the carboxylated CM5 chip surface was activated with 1-Ethyl-3-(3-dimethylaminopropyl) carbodiimide hydrochloride (EDC) and N-hydroxysuccinimide (NHS). The capturing antibody, anti-human IgG(Fc) antibody, was diluted to 25 µg/mL with 10 mM sodium acetate, pH 5.0, and then injected at a flow rate of 10 µL/min to achieve a coupling amount of approximately 10000 response units (RU). Thiswas followed by injection of 1 M ethanolamine to block unreacted groups. For kinetic measurements, the anti-HLA-A24/PRAME monoclonal antibody was diluted into 1 µg/mL, and injected at 10 µL/min to ensure that approximately 60 RU of the antibody was captured by the anti-human Fc antibody. The MIP-P3-His was then serially diluted to graded concentrations (e.g., 0.37 nM, 1.11 nM, 3.33 nM, 10 nM, and 30 nM) and injected at a flow rate of 30 µL/min from lowest to highest concentration, with a binding time of 90 seconds, and a dissociation time of 1200 seconds. The chip surface was regenerated by injecting 3 M of MgCl₂ at a flow rate 10 µL/min for 30 seconds. The association rate (Ka) and the dissociation rate (Kd) were calculated by fitting the association and dissociation sensorgrams to a 1: 1 binding model using Biacore T200 Evaluation Software (version 3.2.1). The dissociation equilibrium constant (K_{D}) was calculated with the K_{d}/Kₐ ratio. The fitting results are shown in Table 1.

**Table 1. Affinity Constant of Anti-HLA-A24/PRAME Antibody Binding to MIP-P3-His**

| | Kₐ (M⁻¹s⁻¹) | K_{d} (s⁻¹) | K_{D} (M) |
|---|---|---|---|
| N83G3 | 6.963E+5 | 4.034E-5 | 5.793E-11 |

### 2.4 Binding of Anti-HLA-A24/PRAME Antibody to PRAME₃₀₁₋₃₀₉ (LYVDSLFFL) Pulsed HT-29 Cells

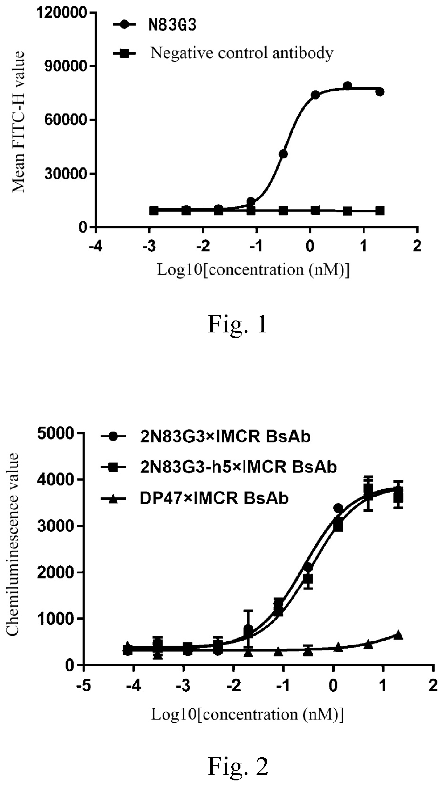

HT-29 cells (human colon cancer cells, purchased from Nanjing Cobioer Biotechnology Co., Ltd.) do not express PRAME protein and have two different HLA-A genes, HLA-A02 and HLA-A24. Log-phase HT-29 cells were collected, digested and centrifuged, then resuspended in RPMI-1640 medium (Gibco, C11875500BT) to a concentration of 1×10⁷ cells/mL, and plated into a 6-well cell culture plate at 1 mL/well. The PRAME₃₀₁₋₃₀₉ peptide (LYVDSLFFL, SEQ ID NO: 19, synthesized by GenScript Biotechnology Co., Ltd.) was diluted to 114 nM with RPMI-1640 medium and added to the 6-well cell culture plate at 1 mL/well, followed by pulsing in a CO₂ incubator at 37°Cfor 3 hours. The pulsed cells were washed once with PBS buffer, resuspended in PBS buffer to a concentration of 2×10⁶ cells/mL, and plated into a 96-well V-bottom plate at 100 µL/well. The test sample, the anti-HLA-A24/PRAME antibody N83G3, and negative control antibody DP47 (prepared according to the Reference ^{[22]}, the amino acid sequences of the heavy chain variable region and the light chain variable region are as set forth in SEQ ID NOs: 96 and 97, respectively) were prepared by diluting with PBS to a starting concentration of 40 nM, with 4-fold dilution gradients for a total of 8 concentrations. 100 µL of the anti-HLA-A24/PRAME antibody N83G3 was added to the cell-containing wells and incubated at 4°C for 1 hour. Then, after washing the wells three times with 200 µL PBS buffer, the cells were incubated with goat anti-human IgG-FITC (Beijing Zhongshan Golden Bridge Biotechnology Co. Ltd., ZF-0308) at 100 µL/well for 30 minutes at 4°C in the dark. After washing the wells three times with 200 µL PBS buffer, the cells were resuspended in100 µL PBS buffer, and detected in the FITC channel by flow cytometry (ACEA, Novocyte). The results show that the anti-HLA-A24/PRAME antibody N83G3 binds well to PRAME₃₀₁₋₃₀₉ pulsed HT-29 cells (FIG. 1).

### Example 3: Humanization and Activity Evaluation of Anti-HLA-A24/PRAME Monoclonal Antibodies

### 3.1 Humanization of Anti-HLA-A24/PRAME Monoclonal Antibodies

The TCRm antibody N83G3 was humanized to reduce its immunogenicity. A classic framework grafting strategy ^{[23]} was used for the humanization scheme. The amino acid sequence of N83G3 was compared to the human antibody germline gene sequences in the IMGT database. Appropriate germline gene sequences were selected to provide the framework regions 1 to 3 of the antibody (FR1+FR2+FR3), and a appropriate J region gene sequence was selected to provide the framework region 4 (FR4). This template can be selected based on a variety of factors, such as the relative total length of the antibody, the size of the CDRs, the amino acid residues at the junction between the antibody framework regions (FRs) and the hypervariable regions (CDRs), the overall sequence homology, and similar considerations. The selected template can be a mixture of multiple sequences or a consensus template to maintain the appropriate conformation of the parental complementarity determining region (CDR) as much as possible. Meanwhile, considering properties such as the solubility, stability, and expression yield of the humanized antibody, four hot-spot amino acids (37F/44E/45R/47F) in its FR2 were reversely mutated to finally obtain the humanized mutant N83G3-h5 (SEQ ID NO: 5).

### 3.2 Affinity Analysis of the Humanized Antibody

Referring to Secotion 2.3, affinity analysis was performed on anti-HLA-A24/PRAME monoclonal antibody N83G3 and its humanized molecule N83G3-h5 using Biacore T200, and the results are shown in Table 2.

**Table 2. Affinity Analysis of Anti-HLA-A24/PRAME Antibody N83G3 and its Humanized Molecule N83G3-h5**

| | Kₐ (M⁻¹s⁻¹) | K_{d} (s⁻¹) | KD (M) |
|---|---|---|---|
| N83G3 | 9.178E+5 | 4.67E-5 | 5.088E-11 |
| N83G3-h5 | 7.368E+5 | 5.521E-5 | 7.474E-11 |

### Example 4: Preparation and Identification of TCRm×CD3 Bispecific Antibody against HLA-A24/PRAME Complex

### 4.1 Preparation of TCRm×CD3 Bispecific Antibody

The nucleotide sequences encoding the divalent N83G3 (2N83G3, SEQ ID NO: 14) variable regions and the nucleotide sequences encoding the anti-CD3 single-chain antibody (IMCR-ScFv, SEQ ID NO: 16, see anti-CD3 v9 of U.S. Pat. No. US 5821337A ^{[24]}) were separately cloned into suitable eukaryotic expression vectors and co-expressed to construct the bispecific antibody against both the HLA-A24/PRAME complex and CD3. Specifically, the nucleotide sequence encoding 2N83G3 was cloned into a eukaryotic expression vector in fusion with the nucleotide sequence encoding the Fc fragment carrying the Hole mutation (IgG1m3-FcH1n1, SEQ ID NO: 33), and the nucleotide sequence encoding IMCR-anti-CD3-ScFv was cloned into a eukaryotic expression vector in fusion with the nucleotide sequence encoding the Fc fragment carrying the Knob mutation (IgG1m3-FcKn1, SEQ ID NO: 34), to construct the bispecific antibody 2N83G3×IMCR.

The eukaryotic expression vectors constructed for expressing 2N83G3-G1m3-FcH1n1 (SEQ ID NO: 35) and IMCR-ScFv-G1m3-FcKn1 (SEQ ID NO: 37) were co-transfected into HEK293F cells using liposomes and cultured under a serum-free suspension culture condition for 3-5 days. The culture supernatant was then collected by centrifugation or the like. The bispecific antibodies in the culture supernatant were purified using Protein A affinity chromatography (e.g., Mabselect SURE from GE Co.), and the recombinant protein preservation buffer was replaced with PBS (pH 7.0) or another suitable buffer using a desalting column (e.g., Hitrap desalting from GE Co.). The desalted protein solution was further purified by size-exclusion chromatography (SEC) using Superdex200 (GE) to obtain the desired protein. If necessary, the antibody samples could be sterilized by filtration and then stored in aliquots at -20°C for future use.

### 4.2 Construction of Humanized TCRm×CD3 Bispecific Antibody against HLA-A24/PRAME Complex

Following the method described in Section 4.1, a nucleotide sequence encoding a divalent N83G3-h5 (2N83G3-h5, SEQ ID NO: 15) variable region and a nucleotide sequence encoding an anti-CD3 single-chain antibody (IMCR-ScFv, SEQ ID NO: 16) were cloned into suitable eukaryotic expression vectors, respectively, and co-expressed to construct a bispecific antibody against HLA-A24/PRAME and CD3. Specifically, the nucleotide sequence encoding 2N83G3-h5 was cloned into a eukaryotic expression vector in fusion with the nucleotide sequence encoding the Fc fragment carrying the Hole mutation (IgG1m3-FcH1n1, SEQ ID NO: 33), and the nucleotide sequence encoding IMCR-ScFv was cloned into a eukaryotic expression vector in fusion with the nucleotide sequence encodingg the Fc fragment carrying the Knob mutation (IgG1m3-FcKn1 (SEQ ID NO: 34), to construct the humanized bispecific antibody 2N83G3-h5×IMCR.

### 4.3 Affinity Analysis of the Humanized Bispecific Antibody

Following the method described in Section 2.3, affinity analysis was performed on the humanized bispecific antibody using Biacore T200, and the results are shown in Tables 3 and 4.

**Table 3. Affinity Constant of the Humanized Bispecific Antibody Binding to MIP-P3-His**

| | Kₐ (M⁻¹s⁻¹) | K_{d} (s⁻¹) | K_{D} (M) |
|---|---|---|---|
| 2N83G3×IMCR | 1.406E+6 | 7.649E-5 | 5.442E-11 |
| 2N83G3-h5×IMCR | 1.241E+6 | 8.064E-5 | 6.498E-11 |

**Table 4. Affinity Constant of the Humanized Bispecific Antibody Binding to CD3**

| | Kₐ (M⁻¹s⁻¹) | K_{d} (s⁻¹) | K_{D} (M) |
|---|---|---|---|
| 2N83G3×IMCR | 5.357E+4 | 2.446E-4 | 4.567E-9 |
| 2N83G3-h5×IMCR | 5.046E+4 | 2.341E-4 | 4.64E-9 |

### 4.4 Humanized Bispecific Antibody Mediates Activation of Jurkat-Dual Cells via Tumor Cells

K562 human chronic myeloid leukemia cells (PRAME-high-expressing, HLA-A24 negative) were purchased from the Cell Resource Center, Institute of Basic Medicine, Chinese Academy of Medical Sciences. The HLA-A24 gene was transfected into K562 cells to construct a K562/HLA-A24 cell strains (PRAME⁺/HLA-A24⁺). Log-phase K562/HLA-A24 cells were collected, resuspended to 5×10⁵ cells/mL with RPMI-1640 medium after centrifugation, and were plated in a 96-well cell plate at 100 µL/well. Log-phase Jurkat-Dual cells (purchased from Invivogen) were collected, centrifuged and resuspended to 1×10⁶ cells/mL with RPMI-1640 medium. Then, the suspension was plated in a 96-well cell plate at 50µL/well to obtain a final E:T ratio of 1: 1. The bispecific antibodies 2N83G3×IMCR BsAb (50 µL/well) or 2N83G3-h5×IMCR BsAb (50 µL/well), or the irrelevant bispecific antibody DP47×IMCR BsAb (50 µL/well), at a starting final concentration of 80 nM (with 4-fold dilution gradients, for a total of 10 concentrations), were then added. After the cell plate was incubated in a CO₂ incubator at 37°C for 20 hours, the supernatant was collected, and the HLA-A24/PRAME×CD3 bispecific antibodies were assayed for their ability to activate Jurkat-Dual cells via tumor cells, according to QUANTI-Luc^{™} instructions (QUANTI-Luc^{™}, Invivogen, rep-qlc2). The results showed that both 2N83G3×IMCR BsAb and 2N83G3-h5×IMCR BsAb could mediate activation of Jurkat-Dual cells via K562/HLA-A24 tumor cells (FIG. 2).

### 4.5 Humanized Bispecific Antibody Mediates Killing of Tumor Cells via PBMCs

### 4.5.1 Intracellular PRAME Expression and HLA-A Locus Typing in Tumor Cells

Six types of cells, including K562 (human chronic myeloid leukemia cells), HT-29 (human colon cancer cells), SK-Hep-1 (human liver cancer cells, purchased from the Cell Resource Center, Institute of Basic Medicine, Chinese Academy of Medical Sciences), MEG-01 (human chronic myelocytic leukemia cells, purchased from Nanjing Cobioer Biotechnology Co., Ltd.), A375 (human malignant melanoma cells, purchased from Nanjing Cobioer Biotechnology Co., Ltd.), and U-2OS (human osteosarcoma cells, purchased from Nanjing Cobioer Biotechnology Co., Ltd.), were respectively detected intracellular PRAME expression by Western blotting (carried out by Beijing Liebake Technology Co., Ltd.), and HLA-A locus typing (carried out by Beijing Boao Jingdian Biotechnology Co., Ltd.). The results are shown in Table 5, indicating that except HT-29 cells, K562, SK-Hep-1, MEG-01, A375 and U-2OS cells all express PRAME, and A375 and U-2OS cells are HLA-A24 negative. That is, only the surfaces of SK-Hep-1, MEG-01 and K562/HLA-A24 cells are HLA-A24⁺/PRAME⁺.

**Table 5. Intracellular PRAME Expression and HLA-A Locus Typing in Tumor Cells**

| Cell Name | PRAME Expression (chemiluminescence signal value) | HLA-A Locus Typing |
|---|---|---|
| K562 | 4438571 | HLA-A11:01 HLA-A31:01 |
| SK-Hep-1 | 91150 | HLA-A02:01 HLA-A24:02 |
| MEG-01 | 118423 | HLA-A24:02 HLA-A24:02 |
| HT-29 | 4446 | HLA-A02:01 HLA-A24:02 |
| A375 | 323344 | HLA-A01:01 HLA-A02:01 |
| U-2OS | 969099 | HLA-A02:01 HLA-A32:01 |

### 4.5.2 Isolation of Human Peripheral Blood Mononuclear Cells (PBMC)

Blood (50 mL) was collected from healthy volunteers who had signed informed consent. The inclusion criteria for volunteers were:
1. Age: ≥ 18 years;
2. No HIV and HBV infection;
3. Normal results of blood routine test; and
4. Non-pregnant or non-lactating female.

PBMC were isolated from the volunteers' whole blood by Ficoll density gradient centrifugation and cultured in RPMI-1640 medium.

### 4.5.3 Humanized Bispecific Antibody 2N83G3-h5×IMCR Mediates Killing of Tumor Cells via PBMCs

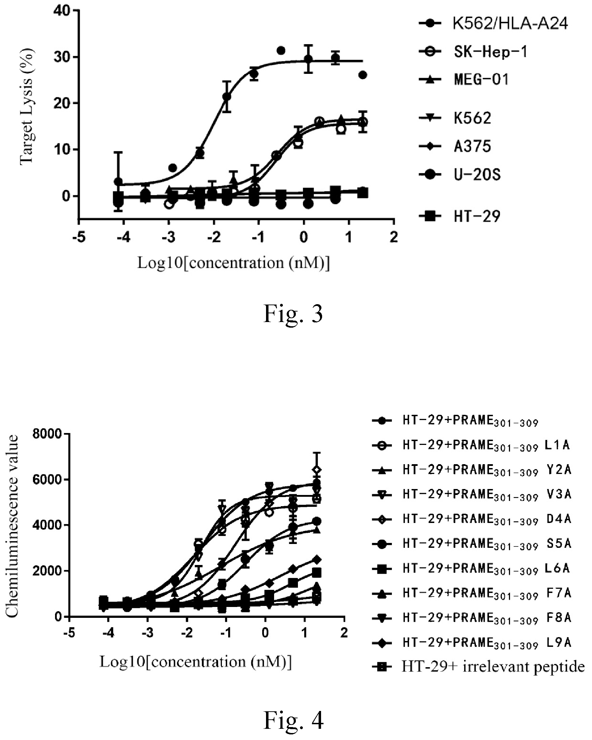

Log-phase K562/HLA-A24 cells, SK-Hep-1 cells, MEG-01 cells, A375 cells, U-2OS cells, HT-29 cells and K562 cells were collected, respectively. The cells were resuspended in RPMI-1640 medium to 5×10⁵ cell/mL after centrifugation, and plated in a 96-well cell plate at 100 µL/well. Then, 4-fold gradient diluted the bispecific antibody 2N83G3-h5×IMCR (50 µL/well) or irrelevant antibody DP47×IMCR (50 µL/well), at a starting concentration of 80 nM (with 4-fold dilution gradients, for a total of 10 concentrations), were added. Finally, 50 µL/well of PBMCs (effector) were added to obtain a final E:T ratio of 5: 1. Meanwhile, the target cells alone controls (K562/HLA-A24 cells, SK-Hep-1 cells, MEG-01 cells, A375 cells, U-2OS cells, HT-29 cells, and K562 cells), the effector cells alone control (PBMCs), a medium alone blank control were set, respectively, using the medium to replenish the volumes to be 200 µL. The cell plate was incubated for 20 hours at 37°C in a CO₂ incubator, then the supernatant was collected, and the killing rate of tumor cells mediated by the bispecific antibody was detected and analyzed according to the instruction of CytoTox 96^{®} Non-Radioactive Cytotoxicity Assay reagent (CytoTox 96^{®} Non-Radioactive Cytotoxicity Assay, Promega, G1780). The results show that the humanized bispecific antibody 2N83G3-h5×IMCR only mediates the killing effect of K562/HLA-A24, SK-Hep-1 and MEG-01 tumor cells via PBMCs, with EC₅₀ values of 0.01234, 0.2405 and 0.2599, respectively (FIG. 3).

### 4.6 Humanized Bispecific Antibody Mediates Activation of Jurkat-Dual Cells via Alanine-substituted Peptide-pulsed HT-29 Cells

Following the method described in Section 2.4, PRAME₃₀₁₋₃₀₉ peptides were substituted with alanine at positions 1, 2, 3, 4, 5, 6, 7, 8 and 9, respectively, to synthesize the corresponding peptides, which were used to pulse HT-29 cells. The amino acid sequence of the irrelevant peptide was as set forth in SEQ ID NO: 98. The results show that the ability of the humanized bispecific antibody 2N83G3-h5×IMCR to mediate the activation of Jurkat-Dual cells via cells pulsed by the peptides substituted with alanine at position 6, 7, 8, or 9 was significantly reduced, and the ability to mediate the activation of Jurkat-Dual cells via cells pulsed by the peptides substituted with alanine at position 4 or 5 was reduced (FIG. 4).

### Example 5: Specificity Validation

### 5.1 Specific Validation of Humanized Bispecific Antibody 2N83G3-h5×IMCR and Similar Peptides of PRAME₃₀₁₋₃₀₉

Cross-reactivity against homologous peptides (sharing key recognition residues) derived from normal tissues might induce severe and not easily predictable toxicities of TCR-like antibodies or TCR-based T-cell therapies (see, e.g., Linette et al., Blood, (2013), 122:863-71, which reported lethal toxicities of MAGE A3-TCR therapies due to off-target reactivity of proteins expressed by cardiac tissue ^{[25]}). Thus, the specificity of these agents is critical. In order to thoroughly validate the specificity of the developed antibodies, based on alanine mutant's data of the PRAME₃₀₁₋₃₀₉ peptide involving in affecting the activity of N83G3-h5 (see, Section 4.6), potential off-target peptides were searched in databases (IEDB and HLA Ligand Atlas) according to the definition formulas xYxxSLFFx, xYxDxLFFx, xYxxxLFFx, xYxxxxFFx, xxxxxLFFx, xYxxSLFxx, xxxDxLFFx, xxxxSLFFx, xxxxxxFFx, and xYxxxxxFx, respectively.A total of 50 peptides derived from different proteins, all of which have high affinity for HLA-A24, were found and synthesized by Genscript Biotech Co., Ltd. Then, cross-reactivity of the humanized bispecific antibody 2N83G3-h5×IMCR was verified using the method of activating Jurkat-Dual cells via peptide-pulsed HT-29 cells, as described in Section 4.6. The results show that the humanized bispecific antibody 2N83G3-h5×IMCR only mediates activation of Jurkat-Dual cells via HT-29 cells peptide pulsed with similar peptides 8/11/12/16, with increasing EC50 values by 337-fold/93-fold/482-fold/97-fold compared with HT-29 cells pulsed with PRAME₃₀₁₋₃₀₉ peptide, respectively. The humanized bispecific antibody 2N83G3-h5×IMCR does not mediate activation of Jurkat-Dual cells via HT-29 cells pulsed with the remaining 46 similar peptides (Table 6).

**Table 6. Similar Peptides of PRAME₃₀₁₋₃₀₉ Peptide**

| Peptide | Amino acid Sequence | Gene Name | EC50 Value (nM) mediating the Activation of Jurkat-Dual Cells | SEQ ID NO. |
|---|---|---|---|---|
| PRAME₃₀₁₋₃₀₉ | LYVDSLFFL | PRAME | 0.01102 | 19 |
| Similar Peptide Segment 1 | SYVRSLPFF | TRAPPC1 | No Activity | 44 |
| Similar Peptide Segment 2 | TYTDRVFFL | Plxnb2 | No Activity | 45 |
| Similar Peptide Segment 3 | TYVDWHFLL | AOC3 | No Activity | 46 |
| Similar Peptide Segment 4 | LYPKTLFLL | PPP3CA | No Activity | 47 |
| Similar Peptide Segment 5 | LYPSTLFLL | PPP3CB | No Activity | 48 |
| Similar Peptide Segment 6 | IYKDSSTFL | METTL14 | No Activity | 49 |
| Similar Peptide Segment 7 | SYVASFFLL | IGSF2 | No Activity | 50 |
| Similar Peptide Segment 8 | VYQHNLFFL | XCR1 | 3.709 | 51 |
| Similar Peptide Segment 9 | YYGPSLFLL | HUWE1 | No Activity | 52 |
| Similar Peptide Segment 10 | LMTDVLVFL | Arhgef2 | No Activity | 53 |
| Similar Peptide Segment 11 | VYTHNLYYL | KIFBP | 1.025 | 54 |
| Similar Peptide Segment 12 | TYNSNLFFW | CPVL | 5.315 | 55 |
| Similar Peptide Segment 13 | AYDDKIYYF | SEMA7A | No Activity | 56 |
| Similar Peptide Segment 14 | AYMPHTFFI | OGT | No Activity | 57 |
| Similar Peptide Segment 15 | AYELTQYYF | TASOR | No Activity | 58 |
| Similar Peptide Segment 16 | IYIENQFFI | PLD1 | 1.073 | 59 |
| Similar Peptide Segment 17 | NYQRLFDFF | MAN2A1 | No Activity | 60 |
| Similar Peptide Segment 18 | RYILEPFFI | SLC1A4 | No Activity | 61 |
| Similar Peptide Segment 19 | KYPLNLYLL | MDS013 | No Activity | 62 |
| Similar Peptide Segment 20 | GYISSAFLF | AOC3 | No Activity | 63 |
| Similar Peptide Segment 21 | TYLLSVYRL | PIK3CG | No Activity | 64 |
| Similar Peptide Segment 22 | VYSNSIYEL | STING1 | No Activity | 65 |
| Similar Peptide Segment 23 | PYISNIYLI | PLOD3 | No Activity | 66 |
| Similar Peptide Segment 24 | KTKSMFFFL | SF3B3 | No Activity | 67 |
| Similar Peptide Segment 25 | DMKTKYFFF | SPCS3 | No Activity | 68 |
| Similar Peptide Segment 26 | NYIDKVRFL | Vimentin | No Activity | 69 |
| Similar Peptide Segment 27 | IYDPNLAFL | PLCG2 | No Activity | 70 |
| Similar Peptide Segment 28 | IYEPNFIFF | SF3B2 | No Activity | 71 |
| Similar Peptide Segment 29 | LYQDKFPFF | ASCC1 | No Activity | 72 |
| Similar Peptide Segment 30 | QYASAFHFL | BBS4 | No Activity | 73 |
| Similar Peptide Segment 31 | KYLEMIYSM | Importin7 | No Activity | 74 |
| Similar Peptide Segment 32 | KYDPNVYSI | Integrin alpha-V | No Activity | 75 |
| Similar Peptide Segment 33 | EYLRQIFRL | SMPD1 | No Activity | 76 |
| Similar Peptide Segment 34 | SYSPQAFKF | LATS1 | No Activity | 77 |
| Similar Peptide Segment 35 | VFLGQAFTI | Derlin-2 | No Activity | 78 |
| Similar Peptide Segment 36 | EYAVMLYTW | CYFIP1 | No Activity | 79 |
| Similar Peptide Segment 37 | YYLPQLYEL | FGD6 | No Activity | 80 |
| Similar Peptide Segment 38 | RYLVTLYGF | TNXB | No Activity | 81 |
| Similar Peptide Segment 39 | TYIKSPPFF | IREB2 | No Activity | 82 |
| Similar Peptide Segment 40 | KYGIVQEFF | SOGA1 | No Activity | 83 |
| Similar Peptide Segment 41 | SYADAFQFL | RTL5 | No Activity | 84 |
| Similar Peptide Segment 42 | VYQLRFQFL | POLR1A | No Activity | 85 |
| Similar Peptide Segment 43 | SYIPSTVFF | TXNL4B | No Activity | 86 |
| Similar Peptide Segment 44 | SYLDSVHFF | EP300 | No Activity | 87 |
| Similar Peptide Segment 45 | IYDFIGEFM | TMEM147 | No Activity | 88 |
| Similar Peptide Segment 46 | YYDLVKAFM | SOS1 | No Activity | 89 |
| Similar Peptide Segment 47 | IYKPVTDFF | LRPPRC | No Activity | 90 |
| Similar Peptide Segment 48 | VYENVSHFL | SLC25A32 | No Activity | 91 |
| Similar Peptide Segment 49 | HYILHNSFF | TIPARP | No Activity | 92 |
| Similar Peptide Segment 50 | AYEIIDQFF | KPNA4 | No Activity | 93 |

### 5.2 Humanized Bispecific Antibody 2N83G3-h5×IMCR Does Not Mediate Killing of HLA-A24 and Similar Peptide Segment Double Positive Cells via PBMCs

### 5.2.1 Construction of PRAME and Similar Peptide Segment 8/11/12/16 Protein Expression Plasmid

The full-length genes of PRAME (SEQ ID NO: 38), XCR1 (similar peptide segment 8, SEQ ID NO: 39), KIFBP (similar peptide segment 11, SEQ ID NO: 40), CPVL (similar peptide segment 12, SEQ ID NO: 41) and PLD1 (similar peptide segment 16, SEQ ID NO: 42) were synthesized according to the UniProt database, and the synthesized recombinant protein genes were cloned into suitable eukaryotic expression vectors using conventional molecular biology techniques to construct expression plasmids based on full-length genes fused with GFP. Similarly, the full-length gene of HLA-A2402 (SEQ ID NO: 43) was synthesized according to the UniProt database, and the synthesized gene was cloned into a suitable eukaryotic expression vector using conventional molecular biology techniques to construct a full-length gene expression plasmid.

### 5.2.2 Humanized Bispecific Antibody 2N83G3-h5×IMCR Mediates Killing of HT-29 Cells Transfected with PRAME or Similar Peptide 8/11/12/16 Gene via PBMCs

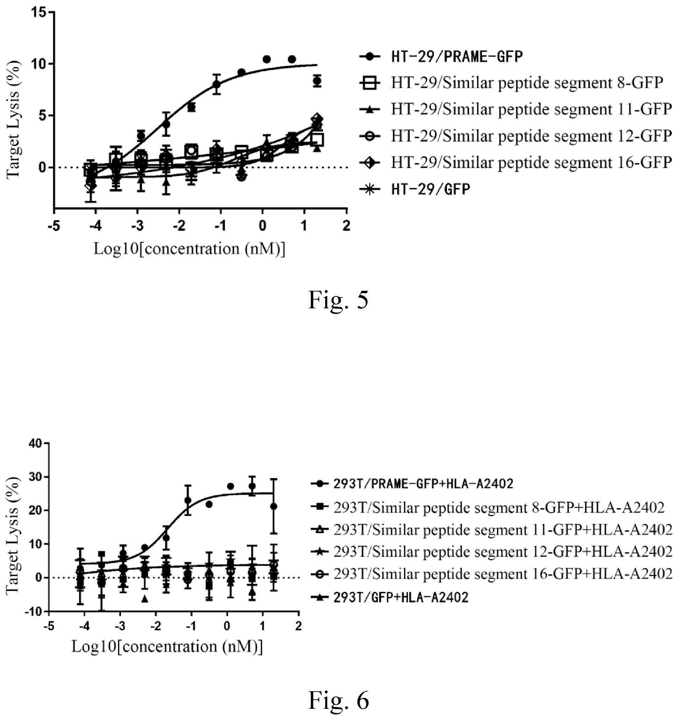

HT-29 cells were plated on the day before transfection, making the cells achieve 50-90% confluence on the day of transfection. That is, Log-phase HT-29 was digested, centrifugated, and then resuspended with RPMI-1640 medium and counted. 1.5×10⁶ of the cells were inoculated into T25 cell culture flasks, respectively, and incubated overnight in a CO₂ incubator at 37°C. On the following day, the PRAME and similar peptide segment 8/11/12/16 gene expression plasmids were respectively transfected into HT-29 cells following the instruction of jetOPTIMUS^{®} transfection reagent (jetOPTIMUS^{®} DNA transfection reagent, Polyplus, 101000006), and the cells were cultured for transfection in a CO₂ incubator at 37°C for 48 hours. Then, the cells were digested, centrifuged and then verified following the method described in Section 4.5. The results show that the humanized bispecific antibody 2N83G3-h5×IMCR mediates only the killing of HT-29 cells transfected with the PRAME gene via PBMCs, and hardly mediates the killing of HT-29 cells respectively transfected with the similar peptide segment 8/11/12/16 gene (FIG. 5).

### 5.2.3 Humanized Bispecific Antibody 2N83G3-h5×IMCR Mediates Killing of 293T Cells Transfected with PRAME or Similar Peptide Segment 8/11/12/16 Genes.

293T cells (human embryonic kidney cells, not expressing PRAME, HLA-A24 negative) were purchased from the Cell Resource Center, Institute of Basic Medicine, Chinese Academy of Medical Sciences). 293T cells were plated on the day before transfection, making the cells achieve 50-90% confluence on the day of transfection. That is, log-phase 293T cells were digested, centrifugated, and then resuspended with PRMI-1640 medium and counted. 1.5×10⁶ of the cells were inoculated into T25 cell culture flasks, respectively, and incubated overnight in a CO₂ incubator at 37°C. On the following day, the HLA-A2402 gene and the PRAME or similar peptide segment 8/11/12/16 gene were co-transfected into 293T cells following the instruction of jetOPTIMUS^{®} transfection reagent (jetOPTIMUS^{®} DNA transfection reagent, Polyplus, 101000006), and the cells were incubated for transfection in a CO₂ incubator at 37°C for 24 hours. Then, the cells were digested, centrifuged, and verified following the method as described in Section 4.5. The results show that the humanized bispecific antibody 2N83G3-h5×IMCR only mediates the killing of 293T cells co-transfected with HLA-A2402+PRAME genes via PBMC, and hardly mediates the killing of 293T cells co-transfected with HLA-A2402+ similar peptide segment 8/11/12/16 gene, respectivley (FIG. 6).

Since the humanized bispecific antibody 2N83G3-h5×IMCR only mediates activation of Jurkat-Dual cells via HT-29 cells pulsed with the similar peptide segment 8/11/12/16 peptide, with increasing EC₅₀ values by about 90-500 folds compared to HT-29 cells pulsed with the PRAME₃₀₁₋₃₀₉ peptide, and can hardly mediate the killing of HT-29 cells transfected with similar peptide segment 8/11/12/16 gene or co-transfected with HLA-A2402+ similar peptide segment 8/11/12/16 gene. Therefore, the TCRm×CD3 bispecific antibody against the HLA-A24/PRAME complex of the present application has specific killing effect on HLA-A24⁺/PRAME⁺ tumors only.

### Example 6: Antitumor Activity of Humanized Bispecific Antibody 2N83G3-h5×IMCR in PBMC Immune Reconstitution Model Mice

Blood (50 mL) was collected from healthy volunteers who had signed informed consent. The inclusion criteria for volunteers were:
1. Age: ≥ 18 years;
2. No HIV and HBV infection;
3. Normal results of blood routine test; and
4. Non-pregnant or non-lactating female.

PBMCs were isolated from healthy human peripheral blood using Ficoll density gradient centrifugation. Forty female NOG mice aged 7-8-week-old (Beijing Vital River Laboratory Animal Technology Co., Ltd.) were selected and inoculated with 1×10⁷ human PBMCs via tail vein. Seven days after human PBMCs inoculation, each mouse was inoculated subcutaneously with 1×10⁷ K562/HLA-A24 cells on the right flank. The inoculation day was defined as day 0. When the average tumor volume reached 110 mm³, the mice were randomly divided into a total of the following four groups based on the tumor volume: bispecific antibody 2N83G3-h5×IMCR (2N83G3-h5×IMCR BsAb) 1 mg/kg group (group 2), bispecific antibody 2N83G3-h5×IMCR 0.1 mg/kg group (group 3), bispecific antibody 2N83G3-h5×IMCR 0.01 mg/kg group (group 4), and bispecific antibody irrelevant antibody DP47×IMCR (DP47×IMCR BsAb) 1.5 mg/kg group (group 1), for eight mice in each group. The mice were administered via tail vein injection at a dosing frequency of once a week for a total of three times. Efficacy was evaluated based on tumor growth volume.

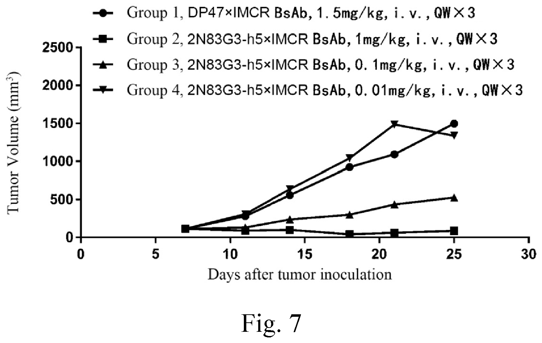

The results show that humanized bispecific antibody 2N83G3-h5×IMCR significantly inhibits tumor growth at dosages of 1 mg/kg and 0.1 mg/kg, with relative tumor growth inhibition TGI (%) of 97.45% and 72.49%, respectively, P < 0.001 (FIG. 7).

### Sequence Listing

**SEQ ID NO: 1**
   IYVMG
**SEQ ID NO: 2**
   VITGSGNTNYADSVKG
**SEQ ID NO: 3**
   GRPSRYYRDSGTWADF
**SEQ ID NO: 4** 
QVQLVESGGGLVQAGGSLRLSCAASGASLDIYVMGWYRQATGKERDLVAVITGSGN
TNYADSVKGRFTISRDDAKNTLYLQMSSLKPEDTAVYYCAGGRPSRYYRDSGTWADFWG
QGTQVTVSS
**SEQ ID NO: 5** 
EVQLVESGGGLVKPGGSLRLSCAASGASLDIYVMGWYRQAPGKGLELVAVITGSGNT
NYADSVKGRFTISRDDSKNTLYLQMNSLKTEDTAVYYCAGGRPSRYYRDSGTWADFWGQ
GTTVTVSS
**SEQ ID NO: 6**
   GYTMN
**SEQ ID NO: 7**
   LINPYKGVSTYNQKFKD
**SEQ ID NO: 8**
   SGYYGDSDWYFDV
**SEQ ID NO: 9**
   RASQDIRNYLN
**SEQ ID NO: 10**
   YTSRLES
**SEQ ID NO: 11**
   QQGNTLPWT
**SEQ ID NO: 12**
**SEQ ID NO: 13**
**SEQ ID NO: 14** QVQLVESGGGLVQAGGSLRLSCAASGASLDIYVMGWYRQATGKERDLVAVITGSGN 
TNYADSVKGRFTISRDDAKNTLYLQMSSLKPEDTAVYYCAGGRPSRYYRDSGTWADFWG
QGTQVTVSSGGGGSGGGGSGGGGSQVQLVESGGGLVQAGGSLRLSCAASGASLDIYVMG
WYRQATGKERDLVAVITGSGNTNYADSVKGRFTISRDDAKNTLYLQMSSLKPEDTAVYYC
AGGRPSRYYRDSGTWADFWGQGTQVTVSS
**SEQ ID NO: 15** 
EVQLVESGGGLVKPGGSLRLSCAASGASLDIYVMGWYRQAPGKGLELVAVITGSGNT
NYADSVKGRFTISRDDSKNTLYLQMNSLKTEDTAVYYCAGGRPSRYYRDSGTWADFWGQ
GTTVTVSSGGGGSGGGGSGGGGSEVQLVESGGGLVKPGGSLRLSCAASGASLDIYVMGW
YRQAPGKGLELVAVITGSGNTNYADSVKGRFTISRDDSKNTLYLQMNSLKTEDTAVYYCA
GGRPSRYYRDSGTWADFWGQGTTVTVSS
**SEQ ID NO: 16** 
DIQMTQSPSSLSASVGDRVTITCRASQDIRNYLNWYQQKPGKAPKLLIYYTSRLESGV
PSRFSGSGSGTDYTLTISSLQPEDFATYYCQQGNTLPWTFGQGTKVEIKGGGGSGGGGSGG
GGSGGGGSGGGSEVQLVESGGGLVQPGGSLRLSCAASGYSFTGYTMNWVRQAPGKGLE
WVALINPYKGVSTYNQKFKDRFTISVDKSKNTAYLQMNSLRAEDTAVYYCARSGYYGDS
DWYFDVWGQGTLVTVSS
**SEQ ID NO: 17** 
QDGNEEMGGITQTPYKVSISGTTVILTCPQYPGSEILWQHNDKNIGGDEDDKNIGSDE
DHLSLKEFSELEQSGYYVCYPRGSKPEDANFYLYLRARVCENCMEMD
**SEQ ID NO: 18**
**SEQ ID NO: 20** 
LYVDSLFFLGCGGSGGGGSGGGGSIQRTPKIQVYSRHPAENGKSNFLNCYVSGFHPSD
IEVDLLKNGERIEKVEHSDLSFSKDWSFYLLYYTEFTPTEKDEYACRVNHVTLSQPKIVKW
DRDMGGGGSGGGGSGGGGSGGGGSGSHSMRYFSTSVSRPGRGEPRFIAVGYVDDTQFVR
FDSDAASQRMEPRAPWIEQEGPEYWDEETGKVKAHSQTDRENLRIALRCYNQSEAGSHT
LQMMFGCDVGSDGRFLRGYHQYAYDGKDYIALKEDLRSWTAADMAAQITKRKWEAAH
VAEQQRAYLEGTCVDGLRRYLENGKETLQRTDPPKTHMTHHPISDHEATLRCWALGFYPA
EITLTWQRDGEDQTQDTELVETRPAGDGTFQKWAAVVVPSGEEQRYTCHVQHEGLPKPLT
LRWE
**SEQ ID NO: 21**
   HHHHHH
**SEQ ID NO: 22** 
ASPRGPTIKPCPPCKCPAPNLLGGPSVFIFPPKIKDVLMISLSPIVTCVVVDVSEDDPDV
QISWFVNNVEVHTAQTQTHREDYNSTLRVVSALPIQHQDWMSGKEFKCKVNNKDLPAPI
ERTISKPKGSVRAPQVYVLPPPEEEMTKKQVTLTCMVTDFMPEDIYVEWTNNGKTELNY
KNTEPVLDSDGSYFMYSKLRVEKKNWVERNSYSCSVVHEGLHNHHTTKSFSRTPGK
**SEQ ID NO: 23** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTVDKSR
 WQQGNVFSCSVMHEALHNHYTQKSLSLSPGK
**SEQ ID NO: 24** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVCTLPPSR
EEMTKNQVSLSCAVKGFYPSDIAVEWESNGQPENNYKTTPPVLDSDGSFFLVSKLTVDKS
RWQQGNVFSCSVMHEALHNHYTQKSLSLSPGK
**SEQ ID NO: 25** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPCR
EEMTKNQVSLWCLVKGFYPSDIAVEWESNGQPENNYKTTPPVLDSDGSFFLYSKLTVDKS
RWQQGNVFSCSVMHEALHNHYTQKSLSLSPGK
**SEQ ID NO: 26** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPEFEGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPASIEKTISKAKGQPREPQVCTLPPSRE
EMTKNQVSLSCAVKGFYPSDIAVEWESNGQPENNYKTTPPVLDSDGSFFLVSKLTVDKSR
WQQGNVFSCSVMHEALHNHYTQKSLSLSPGK
**SEQ ID NO: 27** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVEPKSCDKTHTCPPCPAPEFEGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPASIEKTISKAKGQPREPQVYTLPPCRE
EMTKNQVSLWCLVKGFYPSDIAVEWESNGQPENNYKTTPPVLDSDGSFFLYSKLTVDKSR
WQQGNVFSCSVMHEALHNHYTQKSLSLSPGK
**SEQ ID NO: 28** 
ASTKGPSVFPLAPSSKSTSEGTAALGCLVKDYFPEPVTVSWNSGALTSGVHTFPAVLQ
SSGLYSLSSVVTVPSSSLGTQTYICNVDHKPSNTKVDKTVERKSCVECPPCPAPEFEGGPSV
FLFPPKPKDTLMISRTPEVTCVVVDVSQEDPEVQFNWYVDGVEVHNAKTKPREEQYNST
YRVVSVLTVLHQDWLNGKEYKCKVSNKALPASIEKTISKAKGQPREPQVCTLPPSQEEMT
KNQVSLSCAVKGFYPSDIAVEWESNGQPENNYKTTPPVLDSDGSFFLVSKLTVDKSRWQE
GNVFSCSVMHEALHNHYTQKSLSLSPGK
**SEQ ID NO: 29** 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKRVERKSCVECPPCPAPEFEGGPSV
FLFPPKPKDTLMISRTPEVTCVVVDVSHEDPEVKFNWYVDGVEVHNAKTKPREEQYNST
YRVVSVLTVLHQDWLNGKEYKCKVSNKALPASIEKTISKAKGQPREPQVYTLPPCREEMT
KNQVSLWCLVKGFYPSDIAVEWESNGQPENNYKTTPPVLDSDGSFFLYSKLTVDKSRWQQ
GNVFSCSVMHEALHNHYTQKSLSLSPGK
**SEQ ID NO: 30** 
GQPKAAPSVTLFPPSSEELQANKATLVCLISDFYPGAVTVAWKADSSPVKAGVETTTP
SKQSNNKYAASSYLSLTPEQWKSHRSYSCQVTHEGSTVEKTVAPTECS
**SEQ ID NO: 31**
**SEQ ID NO: 32** 
ASEPKSSDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK
**SEQ ID NO: 33** 
ASERKSSVECPPCPAPEFEGGPSVFLFPPKPKDTLMISRTPEVTCVVVDVSQEDPEVQF
NWYVDGVEVHNAKTKPREEQYNSTYRVVSVLTVLHQDWLNGKEYKCKVSNKALPASIE
KTISKAKGQPREPQVCTLPPSQEEMTKNQVSLSCAVKGFYPSDIAVEWESNGQPENNYKT
TPPVLDSDGSFFLVSKLTVDKSRWQEGNVFSCSVMHEALHNHYTQKSLSLSPGK
**SEQ ID NO: 34** 
ASERKSSVECPPCPAPEFEGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPASIE
KTISKAKGQPREPQVYTLPPCREEMTKNQVSLWCLVKGFYPSDIAVEWESNGQPENNYKT
TPPVLDSDGSFFLYSKLTVDKSRWQQGNVFSCSVMHEALHNHYTQKSLSLSPGK
**SEQ ID NO: 35** 
QVQLVESGGGLVQAGGSLRLSCAASGASLDIYVMGWYRQATGKERDLVAVITGSGN
TNYADSVKGRFTISRDDAKNTLYLQMSSLKPEDTAVYYCAGGRPSRYYRDSGTWADFWG
QGTQVTVSSGGGGSGGGGSGGGGSQVQLVESGGGLVQAGGSLRLSCAASGASLDIYVMG
WYRQATGKERDLVAVITGSGNTNYADSVKGRFTISRDDAKNTLYLQMSSLKPEDTAVYYC
AGGRPSRYYRDSGTWADFWGQGTQVTVSSASERKSSVECPPCPAPEFEGGPSVFLFPPKPK
DTLMISRTPEVTCVVVDVSQEDPEVQFNWYVDGVEVHNAKTKPREEQYNSTYRVVSVLT
VLHQDWLNGKEYKCKVSNKALPASIEKTISKAKGQPREPQVCTLPPSQEEMTKNQVSLSC
AVKGFYPSDIAVEWESNGQPENNYKTTPPVLDSDGSFFLVSKLTVDKSRWQEGNVFSCSV
MHEALHNHYTQKSLSLSPGK
**SEQ ID NO: 36** 
EVQLVESGGGLVKPGGSLRLSCAASGASLDIYVMGWYRQAPGKGLELVAVITGSGNT
NYADSVKGRFTISRDDSKNTLYLQMNSLKTEDTAVYYCAGGRPSRYYRDSGTWADFWGQ
GTTVTVSSGGGGSGGGGSGGGGSEVQLVESGGGLVKPGGSLRLSCAASGASLDIYVMGW
YRQAPGKGLELVAVITGSGNTNYADSVKGRFTISRDDSKNTLYLQMNSLKTEDTAVYYCA
GGRPSRYYRDSGTWADFWGQGTTVTVSSASERKSSVECPPCPAPEFEGGPSVFLFPPKPKD
TLMISRTPEVTCVVVDVSQEDPEVQFNWYVDGVEVHNAKTKPREEQYNSTYRVVSVLTV
LHQDWLNGKEYKCKVSNKALPASIEKTISKAKGQPREPQVCTLPPSQEEMTKNQVSLSCA
VKGFYPSDIAVEWESNGQPENNYKTTPPVLDSDGSFFLVSKLTVDKSRWQEGNVFSCSVM
HEALHNHYTQKSLSLSPGK
**SEQ ID NO: 37** 
DIQMTQSPSSLSASVGDRVTITCRASQDIRNYLNWYQQKPGKAPKLLIYYTSRLESGV
PSRFSGSGSGTDYTLTISSLQPEDFATYYCQQGNTLPWTFGQGTKVEIKGGGGSGGGGSGG
GGSGGGGSGGGSEVQLVESGGGLVQPGGSLRLSCAASGYSFTGYTMNWVRQAPGKGLE
 
WVALINPYKGVSTYNQKFKDRFTISVDKSKNTAYLQMNSLRAEDTAVYYCARSGYYGDS
DWYFDVWGQGTLVTVSSASERKSSVECPPCPAPEFEGGPSVFLFPPKPKDTLMISRTPEVT
CVVVDVSHEDPEVKFNWYVDGVEVHNAKTKPREEQYNSTYRVVSVLTVLHQDWLNGK
EYKCKVSNKALPASIEKTISKAKGQPREPQVYTLPPCREEMTKNQVSLWCLVKGFYPSDIA
VEWESNGQPENNYKTTPPVLDSDGSFFLYSKLTVDKSRWQQGNVFSCSVMHEALHNHYT
QKSLSLSPGK
**SEQ ID NO: 38** 
MERRRLWGSIQSRYISMSVWTSPRRLVELAGQSLLKDEALAIAALELLPRELFPPLFM
AAFDGRHSQTLKAMVQAWPFTCLPLGVLMKGQHLHLETFKAVLDGLDVLLAQEVRPRR
WKLQVLDLRKNSHQDFWTVWSGNRASLYSFPEPEAAQPMTKKRKVDGLSTEAEQPFIPV
EVLVDLFLKEGACDELFSYLIEKVKRKKNVLRLCCKKLKIFAMPMQDIKMILKMVQLDSI
EDLEVTCTWKLPTLAKFSPYLGQMINLRRLLLSHIHASSYISPEKEEQYIAQFTSQFLSLQC
LQALYVDSLFFLRGRLDQLLRHVMNPLETLSITNCRLSEGDVMHLSQSPSVSQLSVLSLSG
VMLTDVSPEPLQALLERASATLQDLVFDECGITDDQLLALLPSLSHCSQLTTLSFYGNSISIS
ALQSLLQHLIGLSNLTHVLYPVPLESYEDIHGTLHLERLAYLHARLRELLCELGRPSMVWL
SANPCPHCGDRTFYDPEPILCPCFMPN
**SEQ ID NO: 39** 
MESSGNPESTTFFYYDLQSQPCENQAWVFATLATTVLYCLVFLLSLVGNSLVLWVLVK
YESLESLTNIFILNLCLSDLVFACLLPVWISPYHWGWVLGDFLCKLLNMIFSISLYSSIFFLTI
MTIHRYLSVVSPLSTLRVPTLRCRVLVTMAVWVASILSSILDTIFHKVLSSGCDYSELTWYL
TSVYQHNLFFLLSLGIILFCYVEILRTLFRSRSKRRHRTVKLIFAIVVAYFLSWGPYNFTLFL
QTLFRTQIIRSCEAKQQLEYALLICRNLAFSHCCFNPVLYVFVGVKFRTHLKHVLRQFWFC
RLQAPSPASIPHSPGAFAYEGASFY
**SEQ ID NO: 40** 
MANVPWAEVCEKFQAALALSRVELHKNPEKEPYKSKYSARALLEEVKALLGPAPED
EDERPEAEDGPGAGDHALGLPAEVVEPEGPVAQRAVRLAVIEFHLGVNHIDTEELSAGEEH
LVKCLRLLRRYRLSHDCISLCIQAQNNLGILWSEREEIETAQAYLESSEALYNQYMKEVGSP
PLDPTERFLPEEEKLTEQERSKRFEKVYTHNLYYLAQVYQHLEMFEKAAHYCHSTLKRQL
EHNAYHPIEWAINAATLSQFYINKLCFMEARHCLSAANVIFGQTGKISATEDTPEAEGEVPE
LYHQRKGEIARCWIKYCLTLMQNAQLSMQDNIGELDLDKQSELRALRKKELDEEESIRKK
AVQFGTGELCDAISAVEEKVSYLRPLDFEEARELFLLGQHYVFEAKEFFQIDGYVTDHIEV
VQDHSALFKVLAFFETDMERRCKMHKRRIAMLEPLTVDLNPQYYLLVNRQIQFEIAHAYY
DMMDLKVAIADRLRDPDSHIVKKINNLNKSALKYYQLFLDSLRDPNKVFPEHIGEDVLRP
AMLAKFRVARLYGKIITADPKKELENLATSLEHYKFIVDYCEKHPEAAQEIEVELELSKEM
VSLLPTKMERFRTKMALT
**SEQ ID NO: 41** 
MVGAMWKVIVSLVLLMPGPCDGLFRSLYRSVSMPPKGDSGQPLFLTPYIEAGKIQKG
RELSLVGPFPGLNMKSYAGFLTVNKTYNSNLFFWFFPAQIQPEDAPVVLWLQGGPGGSSM
FGLFVEHGPYVVTSNMTLRDRDFPWTTTLSMLYIDNPVGTGFSFTDDTHGYAVNEDDVAR
DLYSALIQFFQIFPEYKNNDFYVTGESYAGKYVPAIAHLIHSLNPVREVKINLNGIAIGDGY
SDPESIIGGYAEFLYQIGLLDEKQKKYFQKQCHECIEHIRKQNWFEAFEILDKLLDGDLTSD
PSYFQNVTGCSNYYNFLRCTEPEDQLYYVKFLSLPEVRQAIHVGNQTFNDGTIVEKYLRE
DTVQSVKPWLTEIMNNYKVLIYNGQLDIIVAAALTERSLMGMDWKGSQEYKKAEKKVW
KIFKSDSEVAGYIRQVGDFHQVIIRGGGHILPYDQPLRAFDMINRFIYGKGWDPYVG
**SEQ ID NO: 42**
**SEQ ID NO: 43** 
HSMRYFSTSVSRPGRGEPRFIAVGYVDDTQFVRFDSDAASQRMEPRAPWIEQEGPEY
WDEETGKVKAHSQTDRENLRIALRYYNQSEAGSHTLQMMFGCDVGSDGRFLRGYHQYA
YDGKDYIALKEDLRSWTAADMAAQITKRKWEAAHVAEQQRAYLEGTCVDGLRRYLENG
KETLQRTDPPKTHMTHHPISDHEATLRCWALGFYPAEITLTWQRDGEDQTQDTELVETRPA
GDGTFQKWAAVVVPSGEEQRYTCHVQHEGLPKPLTLRWEPSSQPTIPIVGIIAGLVLFGAVI
TGAVVAAVMWRRKSSDRKGGSYSQAASSDSAQGSDVSLTACKV
**SEQ ID NO: 94**
   GGGGSGGGGSGGGGS
**SEQ ID NO: 95**
   TCCTTCCCCGTCAGCCAGTCCT
**SEQ ID NO: 96** 
EVQLLESGGGLVQPGGSLRLSCAASGFTFSSYAMSWVRQAPGKGLEWVSAISGSGGS
TYYADSVKGRFTISRDNSKNTLYLQMNSLRAEDTAVYYCAKGSGFDYWGQGTLVTVSS
**SEQ ID NO: 97** 
EIVLTQSPGTLSLSPGERATLSCRASQSVSSSYLAWYQQKPGQAPRLLIYGASSRATGI
PDRFSGSGSGTDFTLTISRLEPEDFAVYYCQQYGSSPLTFGQGTKVEIK
**SEQ ID NO: 98**
   TYGPVFMCL

### Reference

[1] Ikeda H, Lethé B, Lehmann F, et al., Characterization of an antigen that is recognized on a melanoma showing partial HLA loss by CTL expressing an NK inhibitory receptor. Immunity. 1997;6(2):199-208.
[2] Wadelin F, Fulton J, McEwan PA, Spriggs KA, Emsley J, Heery DM. Leucine-rich repeat protein PRAME: expression, potential functions and clinical implications for leukaemia. Mol Cancer. 2010; 9:226.
[3] Goodison S, Urquidi V. The cancer testis antigen PRAME as a biomarker for solid tumor cancer management. Biomark Med. 2012;6(5):629-632.
[4] Figueiredo DL, Mamede RC, Proto-Siqueira R, Neder L, Silva WA Jr, Zago MA. Expression of cancer testis antigens in head and neck squamous cell carcinomas. Head Neck. 2006;28(7):614-619.
[5] van't Veer LJ, Dai H, van de Vijver MJ, et al., Gene expression profiling predicts clinical outcome of breast cancer. Nature. 2002;415(6871):530-536.
[6] Neumann E, Engelsberg A, Decker J, et al., Heterogeneous expression of the tumor-associated antigens RAGE-1, PRAME, and glycoprotein 75 in human renal cell carcinoma: candidates for T-cellbased immunotherapies? Cancer Res. 1998; 58(18):4090-4095.
[7] Thongprasert S, Yang PC, Lee JS, et al., The prevalence of expression of MAGE-A3 and PRAME tumor antigens in East and South East Asian non-small cell lung cancer patients. Lung Cancer. 2016; 101:137-144.
[8] van Baren N, Chambost H, Ferrant A, et al., PRAME, a gene encoding an antigen recognized on a human melanoma by cytolytic T cells, is expressed in acute leukaemia cells. Br J Haematol. 1998; 102(5):1376-1379.
[9] Radich JP, Dai H, Mao M, et al., Gene expression changes associated with progression and response in chronic myeloid leukemia. Proc Natl Acad Sci USA. 2006;103(8):2794-2799.
[10] Willenbrock K, Kuppers R, Renne C, et al., Common features and differences in the transcriptome of large cell anaplastic lymphoma and classical Hodgkin's lymphoma. Haematologica. 2006;91(5):596-604.
[11] Doolan P, Clynes M, Kennedy S, Mehta JP, Crown J, O'Driscoll L. Prevalence and prognostic and predictive relevance of PRAME in breast cancer. Breast Cancer Res Treat. 2008;109(2):359-365.
[12] Oberthuer A, Hero B, Spitz R, Berthold F, Fischer M. The tumor-associated antigen PRAME is universally expressed in high-stage neuroblastoma and associated with poor outcome. Clin Cancer Res. 2004;10(13):4307-4313.
[13] Ikeda, H., Lethe, B., Lehmann, F., Van Baren, N., Baurain, J.F., De Smet, C., Chambost, H., Vitale, M., Moretta, A., Boon, T., Coulie, P.G. (1997) Characterization of an antigen that is recognized on a melanoma showing partial HLA loss by CTL expressing an NK inhibitory receptor. Immunity, 6, 199- 208.
[14] A. Margaret Merchant et al., An efficient route to human bispecific IgG, Nature Biotechnology, Volume 16, 1998.
[15]. Kabat, Sequences of Proteins of Immunological Interest, National Institutes of Health, Bethesda, Md. (1991).
[16]. A1-Lazikani et al., J. Mol. Biol. 273:927-948 (1997).
[17]. Martin et al., Proc. Natl. Acad. Sci.USA86:9268-9272 (1989).
[18]. Kotsiou, E., Brzostek, J., Lenart, I., et al., (2010) Dimerization of soluble disulfide trap single-chain major histocompatibility complex class I molecules dependent on peptide binding affinity. Antioxid Redox Signal 15(3):635-644.
[19] Romão, E., Poignavent, V., Vincke, C., et al. (2018) Ritzenthaler C, Muyldermans S, Monsion B. Construction of High-Quality Camel Immune Antibody Libraries. Methods Mol Biol 1701:169-187.
[20] CN 201510097117.0
[21] Phage Display: A Practical Approach / Edited by T. Clackson and H.B. Lowman; Translated by Ma Lan, et al., Chemical Industry Press, May 2008.
[22] US20160200833A1
[23] Tan, P., Mitchell, D, A., Buss, T, N., et al. (2022) "Superhumanized" antibodies: reduction of immunogenic potential by complementarity-determining region grafting with human germline sequences: application to an anti-CD28. J Immunol 169(2):1119-1125.
[24] US 5821337A
[25] Linette et al., Blood, (2013), 122:863-71.

## Claims

1. A bispecific antibody comprising: a first antigen-binding fragment that binds to an HLA-A24/PRAME complex and a second antigen-binding fragment that binds to an activated T cell antigen;
preferably, the first antigen-binding fragment binds to the HLA-A24/PRAME complex at an epitope comprising one or more residues at positions 301-309 of PRAME as set forth in SEQ ID NO: 38;
more preferably, the first antigen-binding fragment binds to the HLA-A24/PRAME complex at an epitope comprising at least one residue at positions 304, 305, 306, 307, 308, and 309 of PRAME as set forth in SEQ ID NO: 38; and/or
preferably, the activated T cell antigen is a CD3 molecule.

2. The bispecific antibody of claim 1, wherein
the first antigen-binding fragment comprises HCDR1 as set forth in SEQ ID NO: 1, HCDR2 as set forth in SEQ ID NO: 2 and HCDR3 as set forth in SEQ ID NO: 3;
preferably, the first antigen-binding fragment is in the form of a single-domain antibody; more preferably, the first antigen-binding fragment comprises a monovalent or multivalent single-domain antibody that binds to the HLA-A24/PRAME complex; and/or
the second antigen-binding fragment comprises HCDR1 as set forth in SEQ ID NO: 6, HCDR2 as set forth in SEQ ID NO: 7, HCDR3 as set forth in SEQ ID NO: 8, LCDR1 as set forth in SEQ ID NO: 9, LCDR2 as set forth in SEQ ID NO: 10, and LCDR3 as set forth in SEQ ID NO: 11;
preferably, the second antigen-binding fragment is in the form of a single-chain variable fragment (scFv) or a Fab fragment; more preferably, the second antigen-binding fragment is in the form of an scFv;
wherein the amino acid sequences of HCDRs are defined according to Kabat numbering scheme.

3. The bispecific antibody of claim 1 or 2, wherein the first antigen-binding fragment comprises a divalent single-domain antibody that binds to the HLA-A24/PRAME complex;
optionally, the first antigen-binding fragment comprises two heavy chain variable regions of monovalent single-domain antibodies that bind to the HLA-A24/PRAME complex;
preferably, the first antigen-binding fragment comprises two heavy chain variable regions of monovalent single-domain antibodies that bind to the HLA-A24/PRAME complex, linked via direct fusion; or two heavy chain variable regions of monovalent single-domain antibodies that bind to the HLA-A24/PRAME complex, linked via a linker;
more preferably, the linker is a GS-type flexible peptide linker; and most preferably, the linker is (G4S)n, (SG4)n or G4(SG4)n, where n is an integer from 1 to 10, such as an integer from 2 to 4.

4. The bispecific antibody of any one of claims 1 to 3, wherein
the first antigen-binding fragment comprises the amino acid sequence as set forth in SEQ ID NO: 4, 5, 14 or 15; and/or
the second antigen-binding fragment comprises a heavy chain variable region having the amino acid sequence as set forth in SEQ ID NO: 12 and a light chain variable region having the amino acid sequence as set forth in SEQ ID NO: 13.

5. The bispecific antibody of any one of claims 1 to 4, wherein the first antigen-binding fragment comprises the amino acid sequence as set forth in SEQ ID NO: 4; and the second antigen-binding fragment comprises a heavy chain variable region having the amino acid sequence as set forth in SEQ ID NO: 12 and a light chain variable region having the amino acid sequence as set forth in SEQ ID NO: 13; or
the first antigen-binding fragment comprises the amino acid sequence as set forth in SEQ ID NO: 5; and the second antigen-binding fragment comprises a heavy chain variable region having the amino acid sequence as set forth in SEQ ID NO: 12 and a light chain variable region having the amino acid sequence as set forth in SEQ ID NO: 13; or
the first antigen-binding fragment comprises the amino acid sequence as set forth in SEQ ID NO: 14; and the second antigen-binding fragment comprises a heavy chain variable region having the amino acid sequence as set forth in SEQ ID NO: 12 and a light chain variable region having the amino acid sequence as set forth in SEQ ID NO: 13; or
the first antigen-binding fragment comprises the amino acid sequence as set forth in SEQ ID NO: 15; and the second antigen-binding fragment comprises a heavy chain variable region having the amino acid sequence as set forth in SEQ ID NO: 12 and a light chain variable region having the amino acid sequence as set forth in SEQ ID NO: 13.

6. The bispecific antibody of any one of claims 1 to 5, wherein
the first antigen binding fragment and the second antigen binding fragment are linked via an Fc fragment of a heavy chain constant region comprising a first Fc fragment and a second Fc fragment;
preferably, the Fc fragment of the heavy chain constant region is an Fc fragment of IgG1 subtype;
more preferably, the Fc fragment of the heavy chain constant region is an Fc fragment of IgG1m3 allotype;
wherein the first Fc fragment comprises the amino acid C at position 354 and the amino acid W at position 366, or the amino acid S at position 349, the amino acid S at position 366, the amino acid A at position 368, and the amino acid V at position 407; and the second Fc fragment comprises the amino acid C at position 354 and the amino acid W at position 366; or the amino acid C at position 349, the amino acid S at position 366, the amino acid A at position 368, and the amino acid V at position 407;
preferably, the first Fc fragment comprises the amino acid C at position 354 and the amino acid W at position 366, and the second Fc fragment comprises the amino acid C at position 349, the amino acid S at position 366, the amino acid A at position 368, and the amino acid V at position 407; and/or
the first Fc fragment and the second Fc fragment comprise the amino acid F at position 234, the amino acid E at position 235 and the amino acid S at position 331, respectively; and/or
one of the first Fc fragment and the second Fc fragment is linked to the first antigen-binding fragment, and the other of the first Fc fragment and the second Fc fragment is linked to the second antigen-binding fragment;
wherein the amino acid positions of the antibody constant regions are determined according to EU numbering scheme.

7. The bispecific antibody of any one of claims 1 to 6, comprising a first arm that binds to the HLA-A24/PRAME complex and a second arm that binds to the activated T cell antigen, wherein
the first arm comprises the amino acid sequence as set forth in SEQ ID NO: 35 or 36; and
the second arm comprises the amino acid sequence as set forth in SEQ ID NO: 37.

8. A nucleic acid molecule encoding the bispecific antibody of any one of claims 1 to 7.

9. A pharmaceutical composition comprising the bispecific antibody of any one of claims 1 to 7, and a pharmaceutically acceptable excipient, diluent or carrier.

10. The pharmaceutical composition of claim 9, for use in the prevention or treatment of an HLA-A24/PRAME-positive tumor;
preferably, the HLA-A24/PRAME-positive tumor is selected from the group consisting of: melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).

11. Use of the bispecific antibody of any one of claims 1 to 7 or the pharmaceutical composition of claim 9 or 10 in the manufacture of a medicament for the prevention or treatment of an HLA-A24/PRAME-positive tumor;
preferably, the HLA-A24/PRAME positive tumor is selected from the group consisting of: melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).

12. A method for preventing or treating a HLA-A24/PRAME-positive tumor, comprising administering to a subject in need thereof the bispecific antibody of any one of claims 1 to 7 or the pharmaceutical composition of claim 9 or 10;
preferably, the HLA-A24/PRAME-positive tumor is selected from the group consisting of: melanoma, non-small cell lung cancer, small cell lung cancer, breast cancer (e.g., triple negative breast cancer), renal cell carcinoma, esophageal cancer, head and neck cancer (e.g., head and neck squamous cell carcinoma), cervical cancer, bladder cancer, hepatocellular carcinoma, gastric cancer, Hodgkin lymphoma, neuroblastoma, acute leukemia (e.g., acute myelogenous leukemia or acute myeloid leukemia), and chronic leukemia (e.g., chronic myelogenous leukemia or chronic myeloid leukemia).
